# EUROPEAN PATENT APPLICATION

(11) **EP 1 849 770 A1**
(43) Date of publication of application: **31.10.2007**
(21) Application number: 06008887.9
(22) Date of filing: 28.04.2006
(51) Int. Cl.: C07D 209/52

(54) **Production process for bicyclic tetrahydropyrrole compounds**

(71) Applicant: LABORATORIOS DEL DR. ESTEVE, S.A., 08026 Barcelona (ES)
(72) Inventor: Pericas-Brondo, Miguel Angel ICIQ, 43007 Tarragona (ES); Torrens-Jover, Antonio, 08221 Tarressa (ES)
(74) Representative: Peters, Hajo

(57) **Abstract**

The present invention relates to a process using the Pauson-Khand Reaction to produce substituted bicyclic tetrahydropyrrole compounds of general formula (I),

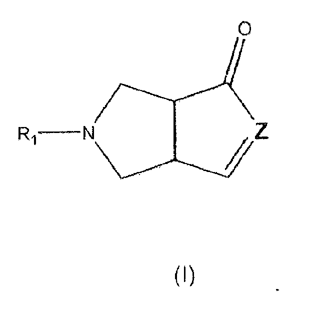

## Description

### Field of the invention

The present invention relates to a production process based on the Pauson-Khand reaction for substituted bicyclic tetrahydropyrrole compounds, especially those having pharmacological activity towards the sigma (σ) receptor.

### Background of the invention

Alzheimer's disease is a progressive, irreversible brain disorder with no known cause or cure. Symptoms of the disease include memory loss, confusion, impaired judgment, personality changes, disorientation, and loss of language skills. Always fatal, Alzheimer's disease is the most common form of irreversible dementia.

According to the American Health Assistance Foundation (AHAF), more than 4.5 million Americans are believed to have Alzheimer's disease and by 2050, the number could increase to 13.2 million. In every nation where life expectancy has increased, so has the incidence of Alzheimer's disease. Alzheimer's disease is becoming tragically common. It is estimated that there are currently 18 million people worldwide with Alzheimer's disease. This figure is projected to nearly double by 2025 to 34 million people.

Considering the fact that there is at present no effective treatment for this fatal disease, it is an imperative to find new solutions to treat AD.

The sigma receptor has at least two subtypes, which may be discriminated by stereoselective isomers of these pharmacoactive drugs. SKF 10047 has nanomolar affinity for the sigma 1 (σ-1) site, and has micromolar affinity for the sigma (σ-2) site. Haloperidol has similar affinities for both subtypes. Endogenous sigma ligands are not known, although progesterone has been suggested to be one of them. Possible sigma-site-mediated drug effects include modulation of glutamate receptor function, neurotransmitter response, neuroprotection, behavior, and cognition (Quirion, R. et al. Trends Pharmacol. Sci., 1992, 13:85-86). Most studies have implied that sigma binding sites (receptors) are plasmalemmal elements of the signal transduction cascade. Drugs reported to be selective sigma ligands have been evaluated as antipsychotics (Hanner, M. et al. Proc. Natl. Acad. Sci., 1996, 93:8072-8077). The existence of sigma receptors in the CNS, immune and endocrine systems have suggested a likelihood that it may serve as link between the three systems.

Therefore, compounds binding to the sigma receptor and which are suitable for modulating these receptors are useful in the prevention and/or the treatment of diseases associated with the sigma receptor.

Recently it has been found that the sigma-1 receptor may be involved in the pathologenesis of Alzheimer's disease (Uchida et al., Am J Geriatr Psychiatry 2005; 13:1062-1066).

Thus, it was an objective of the present invention to provide compounds, especially bicyclic tetrahydropyrrole, by a production process based on the Pauson-Khand reaction to get an elegant way of synthesizing these drugable compounds, especially those that have a pharmacological activity towards the sigma (σ) receptor. These could then be used as active ingredients in medicaments and particularly, these active ingredients should be suitable to modulate the sigma receptor, more particularly the sigma-1 receptor.

Said objective was achieved by providing a production process based on the Pauson-Khand reaction for substituted bicyclic tetrahydropyrrole compounds of general formula (I) given below, their stereoisomers, corresponding salts and corresponding solvates thereof.

Thus, one of the aspect of the present inventon relates to a Process - using the Pauson-Khand Reaction for the production of a substituted bicyclic tetrahydropyrrole compounds of general formula (I) 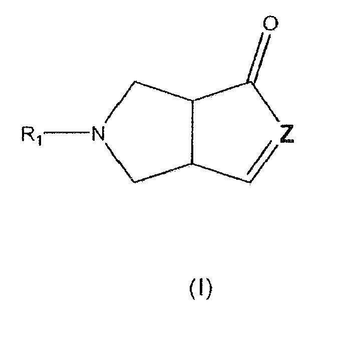 wherein
R¹ represents a hydrogen atom; an unbranched or branched, saturated or unsaturated, optionally at least mono-substituted aliphatic radical; a saturated or unsaturated, optionally at least mono-substituted, optionally at least one heteroatom as ring member containing cyclyl group, which may be condensed with an optionally at least mono-substituted mono- or polycyclic ring system; a branched or unbranched alkyl-cycloalkyl group in which either the alkyl group and/or the cycloalkyl group is optionally at least mono-substituted; a branched or unbranched, saturated or unsaturated, optionally at least mono-substituted alkyl-aryl group in which the aryl group may be condensed with another, optionally at least mono-substituted mono- or polycyclic ring system; a branched or unbranched, saturated or unsaturated, optionally at least mono-substituted alkyl-heterocyclyl group in which the heterocyclyl group is optionally condensed with another, at least mono-substituted mono- or polycyclic ring system; an optionally, at least mono-substituted benzhydryl group; a (C=O)-R² group; a (C=O)-OR³ group; a (SO₂)-R⁴ group; a (C=O)-NR⁵R^{5a} group;
Z represents C-R⁶; C-CHR⁷R^{7a}; a C-(C=O)-R⁸ group; a C-CH₂(SO₂)-R⁹ group; a C-CH₂(SO₂)-NR¹⁰R^{10a} group; or a C-(C=O)-NR¹⁰R^{10a} group;
R², R³, R⁴, and R⁶ represent a hydrogen atom; a linear or branched, saturated or unsaturated, optionally at least mono-substituted aliphatic group; a saturated or unsaturated, optionally at least mono-substituted, optionally at least one heteroatom as ring member containing cyclyl group, which may be condensed with an optionally at least mono-substituted mono- or polycyclic ring system; a branched or unbranched alkyl-cycloalkyl group in which either the alkyl group and/or the cycloalkyl group is optionally at least mono-substituted; a branched or unbranched, saturated or unsaturated, optionally at least mono-substituted alkyl-aryl group in which the aryl group may be condensed with another, optionally at least mono-substituted mono- or polycyclic ring system; a branched or unbranched, saturated or unsaturated, optionally at least mono-substituted alkyl-heterocyclyl group in which the heterocyclyl group is optionally condensed with another, at least mono-substituted mono- or polycyclic ring system;
R⁵ and R^{5a} identical or different, represent a hydrogen atom; a linear or branched, saturated or unsaturated, optionally at least mono-substituted aliphatic group; a saturated or unsaturated, optionally at least mono-substituted, optionally at least one heteroatom as ring member containing cyclyl group, which may be condensed with an optionally at least mono-substituted mono- or polycyclic ring system; a branched or unbranched alkyl-cycloalkyl group in which either the alkyl group and/or the cycloalkyl group is optionally at least mono-substituted; a branched or unbranched, saturated or unsaturated, optionally at least mono-substituted alkyl-aryl group in which the aryl group may be condensed with another, optionally at least mono-substituted mono- or polycyclic ring system; a branched or unbranched, saturated or unsaturated, optionally at least mono-substituted alkyl-heterocyclyl group in which the heterocyclyl group is optionally condensed with another, at least mono-substituted mono- or polycyclic ring system;
R⁷ and R^{7a} identical or different, represent a hydrogen atom; a linear or branched, saturated or unsaturated, optionally at least mono-substituted aliphatic group; an unbranched or branched, saturated or unsaturated, optionally at least mono-substituted alkoxy radical; a saturated or unsaturated, optionally at least mono-substituted, optionally at least one heteroatom as ring member containing cyclyl group, which may be condensed with an optionally at least mono-substituted mono- or polycyclic ring system; a branched or unbranched alkyl-cycloalkyl group in which either the alkyl group and/or the cycloalkyl group is optionally at least mono-substituted; a branched or unbranched, saturated or unsaturated, optionally at least mono-substituted alkyl-aryl group in which the aryl group may be condensed with another, optionally at least mono-substituted mono- or polycyclic ring system; a branched or unbranched, saturated or unsaturated, optionally at least mono-substituted alkyl-heterocyclyl group in which the heterocyclyl group is optionally condensed with another, at least mono-substituted mono- or polycyclic ring system;
R⁸ and R⁹ represent a hydrogen atom; a linear or branched, saturated or unsaturated, optionally at least mono-substituted aliphatic group; an unbranched or branched, saturated or unsaturated, optionally at least mono-substituted alkoxy radical; a saturated or unsaturated, optionally at least mono-substituted, optionally at least one heteroatom as ring member containing cyclyl group, which may be condensed with an optionally at least mono-substituted mono- or polycyclic ring system; a branched or unbranched alkyl-cycloalkyl group in which either the alkyl group and/or the cycloalkyl group is optionally at least mono-substituted; a branched or unbranched, saturated or unsaturated, optionally at least mono-substituted alkyl-aryl group
   in which the aryl group may be condensed with another, optionally at least mono-substituted mono- or polycyclic ring system; a branched or unbranched, saturated or unsaturated, optionally at least mono-substituted alkyl-heterocyclyl group in which the heterocyclyl group is optionally condensed with another, at least mono-substituted mono- or polycyclic ring system;
R¹⁰ and R^{10a}, identical or different, represent a hydrogen atom; a linear or branched, saturated or unsaturated, optionally at least mono-substituted aliphatic group; an unbranched or branched, saturated or unsaturated, optionally at least mono-substituted alkoxy radical; a saturated or unsaturated, optionally at least mono-substituted, optionally at least one heteroatom as ring member containing cyclyl group, which may be condensed with an optionally at least mono-substituted mono- or polycyclic ring system; a branched or unbranched alkyl-cycloalkyl group in which either the alkyl group and/or the cycloalkyl group is optionally at least mono-substituted; a branched or unbranched, saturated or unsaturated, optionally at least mono-substituted alkyl-aryl group in which the aryl group may be condensed with another, optionally at least mono-substituted mono- or polycyclic ring system; a branched or unbranched, saturated or unsaturated, optionally at least mono-substituted alkyl-heterocyclyl group in which the heterocyclyl group is optionally condensed with another, at least mono-substituted mono- or polycyclic ring system;
optionally in form of one of the stereoisomers, preferably enantiomers or diastereomers, a racemate or in form of a mixture of at least two of the stereoisomers, preferably enantiomers and/or diastereomers, in any mixing ratio, or a corresponding salt thereof, or a corresponding solvate thereof;
wherein one substituted pyrroline compound of general formula (II) 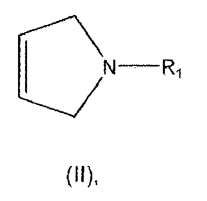 wherein R¹ has the meaning given above, is reacted - using a Pauson-Khand additive at any time during this reaction - with an acetylene compound of general formula (III), 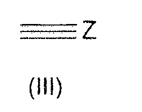 wherein Z has the meaning given above, to give compounds of general formula (I), 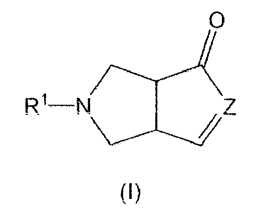 in which R¹ and Z have the meaning given above.

In a preferred embodiment of the present invention the following proviso applies to the compounds of general formula (I)
if Z represents CH, R¹ may not represent a benzyl group.

The Pauson-Khand reaction is a well-known chemical reaction known from textbooks to anyone skilled in the art. References include the following which are all included by reference into this description:
- Coordination Chemistry Reviews, 188 (1999), 297-341;
- Tetrahedron, 56 (2000), 3263-3283;
- Synlett 1999, 6, 768-770;
- Chem. Eur. J. 2001, 7 (8),1589-1595;
- Journal of Organometallic Chemistry, 354 (1988) 233-242;
- Synlett 1999, 6, 771-773;
- Org. Biomol. Chem., 2005, 3, 2396-2398.

Nevertheless the Pauson-Khand Reaction was never used for above-described synthesis and surprisingly gave a highly elegant way of synthesizing the (new) compounds of general formula I.

Unless otherwise stated, the compounds synthesized according to the invention are also meant to include compounds which differ only in the presence of one or more isotopically enriched atoms. For example, compounds having the present structures except for the replacement of a hydrogen by a deuterium or tritium, or the replacement of a carbon by ¹³C- or ¹⁴C-enriched carbon or ¹⁵N-enriched nitrogen are within the scope of this invention.

The term "condensed" according to the present invention means that a ring or ring-system is attached to another ring or ring-system, whereby the terms "annulated" or "annelated" are also used by those skilled in the art to designate this kind of attachment.

The term "ring system" according to the present invention refers to ring sytems comprises saturated, unsaturated or aromatic carbocyclic ring sytems which contain optionally at least one heteroatom as ring member and which are optionally at least mono-substituted. Said ring systems may be condensed to other carbocyclic ring systems such as aryl groups, naphtyl groups, heteroaryl groups, cycloalkyl groups, etc.

Cyclyl groups/radicals, as defined in the present invention, comprise any saturated, unsaturated or aromatic carbocyclic ring sytems which contain optionally at least one heteroatom as ring member and which are optionally at least mono-substituted. Cyclyl groups preferably comprise aryl, heteroaryl, cyclyl, heterocylcyl and/or spiro ring systems.

Heterocyclyl groups/radicals, as defined in the present invention, comprise any saturated, unsaturated or aromatic carbocyclic ring sytems which are optionally at least mono-substituted and which contain at least one heteroatom as ring member. Preferred heteroatoms for these heterocyclyl groups are N, S or O.

Aliphatic radicals/groups, as referred to in the present invention, are optionally mono-or polysubstituted and may be branched or unbranched, saturated or unsaturated. Unsaturated aliphatic groups, as defined in the present invention, include alkyl, alkenyl and alkinyl radicals. Preferred aliphatic radicals according to the present invention include but are not restricted to methyl, ethyl, vinyl (ethenyl), ethinyl, propyl, n-propyl, isopropyl, allyl (2-propenyl), 1-propinyl, methylethyl, butyl, n-butyl, iso-butyl, sec-butyl, tert-butyl butenyl, butinyl, 1-methylpropyl, 2-methylpropyl, 1,1-dimethylethyl, pentyl, n-pentyl, 1,1-dimethylpropyl, 1,2-dimethylpropyl, 2,2-dimethylpropyl, hexyl, 1-methylpentyl, n-heptyl, n-octyl, n-nonyl and n-decyl. Preferred substituents for aliphatic radicals, according to the present invention, are a C₁₋₄ alkyl group, a linear or branched C₁₋₆ alkoxy group, F, Cl, I, Br, CF₃, CH₂F, CHF₂, CN, OH, SH, NH₂, oxo, (C=O)R', SR', SOR', SO₂R', NHR', NR'R" whereby R' and optionally R" for each substitutent independently represents a linear or branched C₁₋₆-alkyl group.

Alkyl radicals, as referred to in the present invention, are saturated aliphatic radicals. They may be linear or branched and are optionally substituted.

Cycloalkyl radicals, as referred to in the present invention, are understood as meaning saturated and unsaturated (but not aromatic), cyclic hydrocarbons, which can optionally be unsubstituted, mono- or polysubstituted. In these radicals, for example C₃₋₄-cycloalkyl represents C₃- or C₄-cycloalkyl, C₃₋₅-cycloalkyl represents C₃-, C₄- or C₅-cycloalkyl, etc. With respect to cycloalkyl, the term also includes saturated cycloalkyls in which optionally at least one carbon atom may be replaced by a heteroatom, preferably S, N, P or O. However, mono- or polyunsaturated, preferably monounsaturated, cycloalkyls without a heteroatom in the ring also in particular fall under the term cycloalkyl as long as the cycloalkyl is not an aromatic system.

Examples for cycloalkyl radicals preferably include but are not restricted to cyclopropyl, 2-methylcyclopropyl, cyclopropylmethyl, cyclobutyl, cyclopentyl, cyclopentylmethyl, cyclohexyl, cycloheptyl, cyclooctyl, acetyl, tert-butyl, adamantyl, pyrroline, pyrrolidine, pyrrolidineone, pyrazoline, pyrazolinone, oxopyrazolinone, aziridine, acetidine, tetrahydropyrrole, oxirane, oxetane, dioxetane, tetrahydrofurane, dioxane, dioxolane, oxathiolane, oxazolidine, thiirane, thietane, thiolane, thiane, thiazolidine, piperidine, piperazine or morpholine.

Cycloalkyl radicals, as defined in the present invention, are optionally mono-or polysubstituted by substitutents independently selected from a C₁₋₄ alkyl group, a linear or branched C₁₋₆ alkoxy group, F, Cl, I, Br, CF₃, CH₂F, CHF₂, CN, OH, SH, NH₂, oxo, (C=O)R', SR', SOR', SO₂R', NHR', NR'R" whereby R' and optionally R" for each substitutent independently represents a linear or branched C₁₋₆-alkyl group.

An aryl radical, as referred to in the present invention, is understood as meaning ring systems with at least one aromatic ring but without heteroatoms even in only one of the rings. These aryl radicals may optionally be mono-or polysubstituted by substitutents independently selected from a C₁₋₄ alkyl group, a linear or branched C₁₋₆ alkoxy group, an optionally at least mono-substituted phenyl group, F, Cl, I, Br, CF₃, CH₂F, CHF₂, CN, OH, SH, NH₂, OXO, (C=O)R', SR', SOR', SO₂R', N(C=O)-OR', NHR', NR'R" whereby R' and optionally R" for each substitutent independently represents a linear or branched C₁₋₆-alkyl group. Preferred examples of aryl radicals include but are not restricted to phenyl, naphthyl, fluoranthenyl, fluorenyl, tetralinyl or indanyl or anthracenyl radicals, which may optionally be mono- or polysubstituted, if not defined otherwise.

An alkyl-aryl radical, as defined in the present invention, comprises a linear or branched, optionally at least mono-substituted alkyl chain which is bonded to an aryl group, as defined above. A preferred alkyl-aryl radical is a benzyl group, wherein the alkyl chain is optionally branched or substituted. Preferred substituents for alky-aryl radicals, according to the present invention, are F, Cl, Br, I, NH₂, SH, OH, SO₂, CF₃, carboxy, amido, cyano, carbamyl, nitro, phenyl, benzyl, -SO₂NH₂ C₁₋₆ alkyl and/or C₁₋₆-alkoxy.

A heteroaryl radical is understood as meaning heterocyclic ring systems which have at least one aromatic ring and may optionally contain one or more heteroatoms from the group consisting of nitrogen, oxygen and/or sulfur and may optionally be mono-or polysubstituted by substitutents independently selected from a C₁₋₄ alkyl group, a linear or branched C₁₋₆ alkoxy group, F, Cl, I, Br, CF₃, CH₂F, CHF₂, CN, OH, SH, NH₂, oxo, (C=O)R', SR', SOR', SO₂R', NHR', NR'R" whereby R' and optionally R" for each substitutent independently represents a linear or branched C₁₋₆-alkyl group. Preferred examples of heteroaryls include but are not restricted to furan, benzofuran, thiophene, benzothiophene, pyrrole, pyridine, pyrimidine, pyridazine, pyrazine, quinoline, isoquinoline, phthalazine, benzo-1,2,5-thiadiazole, benzothiazole, indole, benzotriazole, benzodioxolane, benzodioxane, benzimidzole, carbazole and quinazoline.

A general scheme of the reaction to the intermediate (the 1-substituted-3-pyrrolines) to compounds of general formula (I) - via a Pauson-Khand reaction - is given below in scheme (I):

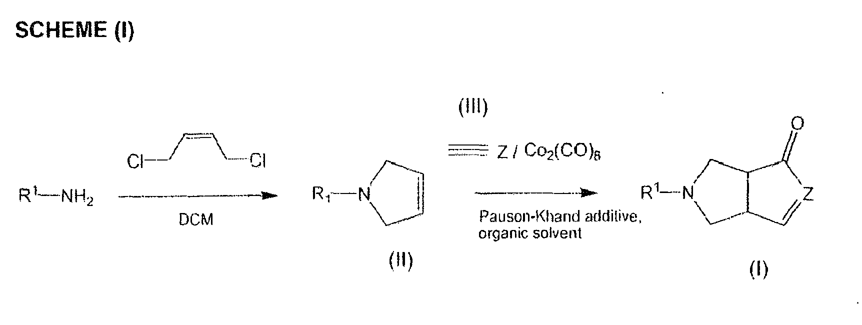

The synthesis of 1-substituted-3-pyrrolines of general formula (II) is well known by those skilled in the art and is described in e.g. JP2001278857, JP2001270862, Synthetic Communications 1990, 20(2), 227-230, Synthetic Communications 2004, 34(23), 4421 or JP2005120067.

Compounds of general formula (Ib) can be obtained by performing a 1,4-addition reaction with a compound of general formula (I), 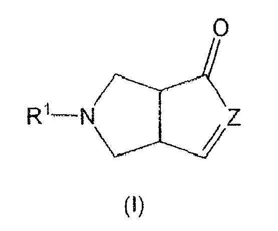 wherein R¹ and Z have the meaning as described above, to give a compound of general formula (Ib), 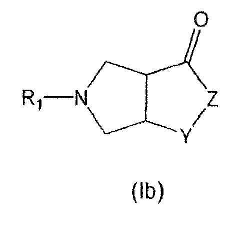 wherein R¹ and Z have the meaning as defined above, and Y represents a CH₂ group; a C-R¹¹R¹² group; a CH-(C=O)-R¹⁶ group; a CH-(SO₂)-R¹⁷ group; CH-(SO₂)-NR¹⁸R^{18a} group; or a CH-(C=O)-NR¹⁸R^{18a} group;
R¹¹ and R¹², identical or different, represent represent a hydrogen atom; a linear or branched, saturated or unsaturated, optionally at least mono-substituted aliphatic group; an unbranched or branched, saturated or unsaturated, optionally at least mono-substituted alkoxy radical; a saturated or unsaturated, optionally at least mono-substituted, optionally at least one heteroatom as ring member containing cyclyl group, which may be condensed with an optionally at least mono-substituted mono- or polycyclic ring system; a branched or unbranched alkyl-cycloalkyl group in which either the alkyl group and/or the cycloalkyl group is optionally at least mono-substituted; a branched or unbranched, saturated or unsaturated, optionally at least mono-substituted alkyl-aryl group in which the aryl group may be condensed with another, optionally at least mono-substituted mono- or polycyclic ring system; a branched or unbranched, saturated or unsaturated, optionally at least mono-substituted alkyl-heterocyclyl group in which the heterocyclyl group is optionally condensed with another, at least mono-substituted mono- or polycyclic ring system; a -(SO₂)-R¹³-group; or a -NR¹⁴R¹⁵-group;
R¹³, R¹⁴, R¹⁵, R¹⁶ and R¹⁷ represent a hydrogen atom; a linear or branched, saturated or unsaturated, optionally at least mono-substituted aliphatic group; an unbranched or branched, saturated or unsaturated, optionally at least mono-substituted alkoxy radical; a saturated or unsaturated, optionally at least mono-substituted, optionally at least one heteroatom as ring member containing cyclyl group, which may be condensed with an optionally at least mono-substituted mono- or polycyclic ring system; a branched or unbranched alkyl-cycloalkyl group in which either the alkyl group and/or the cycloalkyl group is optionally at least mono-substituted; a branched or unbranched, saturated or unsaturated, optionally at least mono-substituted alkyl-aryl group in which the aryl group may be condensed with another, optionally at least mono-substituted mono- or polycyclic ring system; a branched or unbranched, saturated or unsaturated, optionally at least mono-substituted alkyl-heterocyclyl group in which the heterocyclyl group is optionally condensed with another, at least mono-substituted mono- or polycyclic ring system;
R¹⁸ and R^{18a}, identical or different, represent a hydrogen atom; a linear or branched, saturated or unsaturated, optionally at least mono-substituted aliphatic group; an unbranched or branched, saturated or unsaturated, optionally at least mono-substituted alkoxy radical; a saturated or unsaturated, optionally at least mono-substituted, optionally at least one heteroatom as ring member containing cyclyl group, which may be condensed with an optionally at least mono-substituted mono- or polycyclic ring system; a branched or unbranched alkyl-cycloalkyl group in which either the alkyl group and/or the cycloalkyl group is optionally at least mono-substituted; a branched or unbranched, saturated or unsaturated, optionally at least mono-substituted alkyl-aryl group in which the aryl group may be condensed with another, optionally at least mono-substituted mono- or polycyclic ring system; a branched or unbranched, saturated or unsaturated, optionally at least mono-substituted alkyl-heterocyclyl group in which the heterocyclyl group is optionally condensed with another, at least mono-substituted mono- or polycyclic ring system;
optionally in form of one of the stereoisomers, preferably enantiomers or diastereomers, a racemate or in form of a mixture of at least two of the stereoisomers, preferably enantiomers and/or diastereomers, in any mixing ratio, or a corresponding salt thereof, or a corresponding solvate thereof.

The performance of said 1,4-addition reaction is well known by those skilled in the art and is preferably done in the presence of a catalyst such as Copper iodide and an apolar substrate such as e.g. Et₂O. The reactants in this 1,4-addition may be metallic or non-metallic. Preferably, the reactants are metallic.

Preferred examples of metallic reactants are Y-Li and Y-Mgₓ, wherein Y represents a CH₂ group; a C-R¹¹R¹² group; a CH-(C=O)-R¹⁶ group; a CH-(SO₂)-R¹⁷ group; CH-(SO₂)-NR¹⁸R^{18a} group; or a CH-(C=O)-NR¹⁸R^{18a} group; and x refers to the valency of Mg ,depending on the ligand Y.

A general scheme for compounds of general formula (Ib) with Y and Z forming a saturated bond (Y-Z) is given below in scheme (II);

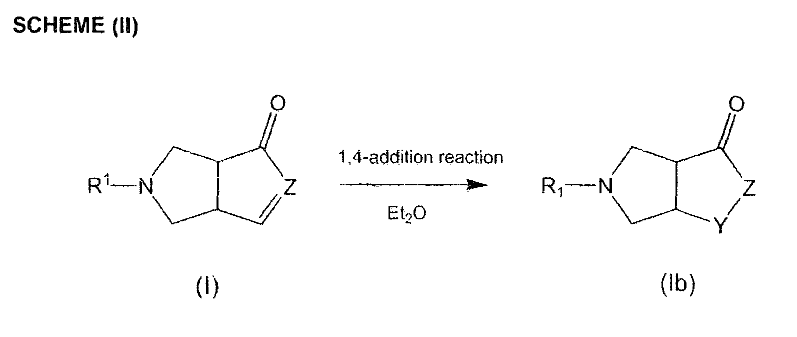

During the processes described above the protection of sensitive groups or of reagents may be necessary and/or desirable. The introduction of conventional protective groups as well as their removal may be performed by methods well-known to those skilled in the art.

If the compounds of general formula (I) themselves are obtained in form of a mixture of stereoisomers, particularly enantiomers or diastereomers, said mixtures may be separated by standard procedures known to those skilled in the art, e.g. chromatographic methods or fractionalized crystallization with chiral reagents. If there are chiral centers the compounds may be prepared in racemic form, or individual enantiomers may be prepared either by enantiospecific synthesis or by resolution. Solvates, preferably hydrates, of the compounds of general formula (I), of corresponding stereoisomers, or of corresponding salts thereof may also be obtained by standard procedures known to those skilled in the art.

The purification and isolation of the inventive compounds of general formula (I), of a corresponding stereoisomer, or salt, or solvate or any intermediate thereof may, if required, be carried out by conventional methods known to those skilled in the art, e.g. chromatographic methods or recrystallization.

Preferred is a process according to the invention, wherein
- the reaction is done in an organic solvent and/or
- the reaction temperature is between 20° C and 120° C, preferably between 60° C and 100° C, most preferably between 75° C and 95° C and/or
- the reaction time is between 1h and 48h, preferably between 12h and 24h and/or
- Co₂(CO)₈ is added at any time during this reaction.

Highly preferred is a process according to the invention, wherein in a preceeding
step A) in an organic solvent a compound of general formula (III), 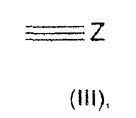 (wherein Z has the meaning as described above), and Co₂(CO)₈ are dissolved/suspended and stirred between 0.5h and 4h at a temperature between 4° C and 100° C, then in a
step B) a compound according to general formula II 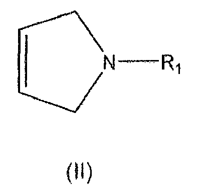 (wherein R¹ has the meaning as described above) is added together with a Pauson-Khand additive and stirred for between 1 h and 48 h at between 20° C to 100°C.

Highly preferred is a process according to the invention, wherein the compound according to general formula II and/or the Pauson-Khand additive is dissolved in an organic solvent before adding in Step B) to the compound arcording to general formula lll.

Highly preferred is a process according to the invention, wherein the organic solvent is halogenated and/or aliphatic, preferably is halogenated and aliphatic, most preferably is 1,2-dichloroethane.

Highly preferred is a process according to the invention, wherein in step A)
- the reaction temperature is between 10° C and 40° C, preferably between 20° C and 30° C, most preferably at room temperature and/or
- the reaction time is between 0.75 h and 12 h, preferably between 1 h and 4h, most preferably between 1.5h and 2.5 h.

Highly preferred is a process according to the invention, wherein in step B)
- the reaction temperature is between 50° C and 120° C, preferably between 60° C and 90° C, most preferably at between 75° C and 85° C or at room temperature and/or
- the reaction time is between 6h and 48h, preferably between 12h and 36h, most preferably between 18h and 24h.

Highly preferred is a process according to the invention, wherein the compound according to general formula III and the compound according to general formula II are reacted in equal molar amounts ± 20%.

Highly preferred is a process according to the invention, wherein the compound according to general formula III, the compound according to general formula II and Co₂(CO)₈ are reacted in equal molar amounts ± 20%.

Highly preferred is a process according to the invention, wherein the Pauson-Khand additive is selected from
- Amine N-oxides (R₃NO)
- 4-methylmorpholine N-oxide
- Trimethylamine N-oxide
- Amines
- Cyclohexylamine
- Benzylamine
- α-methylbenzylamine
- Cyclooctylamine
- Phosphine-oxides (R₃PO)
- Tributylphosphine-oxide
- Sulphoxides (R-SO-R)
- Dimethylsulphoxide
- (-)-methyl-p-tolylsulfoxide
- Dialkylsulfides (R-S-R)
- n-butyl methyl sulphide
- methyl phenyl sulphide
- tert-butyl methyl sulphide
- dodecyl methyl sulphide
- 4-methylmorpholine N-oxide
- Methyl phenyl sulphide
- Cyclohexylamine
- Dimethylsulphoxide
- Trioctyl phosphine oxide
- Cyclopentylamine
- Trans-1,2-diaminocyclohexane
- 2-aminobutanol;
preferably from
- 4-methylmorpholine N-oxide
- Trimethylamine N-oxide
- Cyclohexylamine
- Benzylamine
- α-methylbenzylamine
- Cyclooctylamine
- Tributylphosphine-oxide
- Dimethylsulphoxide
- (-)-methyl-p-tolylsulfoxide
- n-butyl methyl sulphide
- methyl phenyl sulphide
- tert-butyl methyl sulphide
- dodecyl methyl sulphide
- 4-methylmorpholine N-oxide
- Methyl phenyl sulphide
- Cyclohexylamine
- Dimethylsulphoxide
- Trioctyl phosphine oxide
- Cyclopentylamine;
more preferably from
- 4-methylmorpholine N-oxide
- Methyl phenyl sulphide
- Cyclohexylamine
- Dimethylsulphoxide
- Trioctyl phosphine oxide
- Cyclopentylamine;
most preferably
- Cyclohexylamine
- Dimethylsulphoxide
- Trioctyl phosphine oxide
- Cyclopentylamine.

### Additives in Pauson-Khand reaction.

The following additives have been used as promoters in the Pauson-Khand reactions as described in the art. (For reviews in this topic see for example: Coordination Chemistry Reviews, 188 (1999), 297-341. Tetrahedron, 56 (2000), 3263-3283)

### Amine N-oxides (R₃NO)

### Examples:

4-methylmorpholine N-oxide
Trimethylamine N-oxide

### Amines

### Examples:

Cyclohexylamine
Benzylamine
α-methylbenzylamine
Cyclooctylamine
And others...
(see for example: Synlett 1999, 6, 768-770. Chem. Eur.J. 2001, 7 (8), 1589-1595.)

### Phosphine-oxides (R₃PO)

Tributylphosphine-oxide (in ultrasonic irradiation conditions)
(Ref. Journal of Organometallic Chemistry, 354 (1988) 233-242.

### Sulphoxides (R-SO-R)

### Dimethylsulphoxide

### (-)-methyl-p-tolylsulfoxide

### Dialkylsulfides (R-S-R)

### Examples:

n-butyl methyl sulphide
methyl phenyl sulphide
tert-butyl methyl sulphide
dodecyl methyl sulphide
and others...
(see Synlett 1999, 6, 771-773. Org. Biomol. Chem., 2005, 3, 2396-2398.

Most highly preferred is the synthesis of compounds of general formula (I) as described above, selected from the group consisting of:
[1] 2-benzyl-5-phenyl-1,2,3,3a-tetrahydrocyclopenta[*c*]pyrrol-4(6a*H*)-one,
[2] 2-benzyl-5-phenyl-1,2,3,3a-tetrahydrocyclopenta[*c*]pyrrol-4(6a*H*)-one,
[3] 2-(4-methoxybenzyl)-5-phenyl-1,2,3,3a-tetrahydrocyclopenta[*c*]pyrrol-4(6a*H*)-one,
[4] 2-benzyl-5-(4-fluorophenyl)-1,2,3,3a-tetrahydrocyclopenta[*c*]pyrrol-4(6a*H*)-one,
[5] 2-benzyl-5-(4-ethylphenyl)1,2,3,3a-tetrahydrocyclopenta[*c*]pyrrol-4(6a*H*)-one,
[6] 2-benzyl-5-(2-chlorophenyl)-1,2,3,3a-tetrahydrocyclopenta[*c*]pyrrol-4(Ga*H*)-one,
[7] 2-benzyl-5-(4-chlorophenyl)-1,2,3,3a-tetrahydrocyclopenta[*c*]pyrrol-4(6a*H*)-one,
[8] 2-benzyl-5-(3-chlorophenyl)-1,2,3,3a-tetrahydrocyclopenta[*c*]pyrrol-4(6a*H*)-one,
[9] 2-benzyl-5-(4-methoxyphenyl)-1,2,3,3a-tetrahydrocyclopenta[*c*]pyrrol-4(6a*H*]-one,
[10] 2-benzyl-5-(biphenyl-4-yl)-1,2,3,3a-tetrahydrocyclopenta[*c*]pyrrol-4(6a*H*)-one,
[11] 2-benzyl-5-(4-*tert*-butylcarbamatephenyl)-1,2,3,3a-tetrahydrocyclopenta[c]pyrrol-4(6aH)-one,
[12] 2-benzyl-5-butyl-1,2,3,3a-tetrahydrocyclopenta[*c*]pyrrol-4(6a*H*]-one,
[13] 2-benzhydryl-5-phenyl-1,2,3,3a-tetrahydrocyclopenta[*c*]pyrrol-4(6a*H*)-one,
[14] 2,5-dibenzyl-1,2,3,3a-tetrahydrocyclopenta[*c*]pyrrol-4(6a*H*)-one,
[15] 2-(4-fluorobenzyl)-5-phenyl-1,2,3,3a-tetrahydrocyclopenta[*c*]pyrrol-4(6a*H*)-one,
[16] 2-benzyl-6-*tert*-butyl-1,2,3,3a-tetrahydrocyclopenta[*c*]pyrrol-4(6a*H*)-one,
optionally in form of one of the stereoisomers, preferably enantiomers or diastereomers, a racemate or in form of a mixture of at least two of the stereoisomers, preferably enantiomers and/or diastereomers, in any mixing ratio, or a corresponding salt thereof, or a corresponding solvate thereof.

Many of the compounds of general formula (I) and given below, stereoisomers thereof, corresponding salts and corresponding solvates have high affinity to sigma receptors, i.e. they are selective ligands for the sigma receptor and act as modulators, e.g. antagonists, inverse agonists or agonists, on these receptors.

These compounds of general formula (I) given below, their stereoisomers, corresponding salts thereof and corresponding solvates are toxicologically acceptable and are therefore suitable as pharmaceutical active substances for the preparation of medicaments.

One preferred pharmaceutically acceptable form is the crystalline form, including such form in pharmaceutical composition. In the case of salts and solvates the additional ionic and solvent moieties must also be non-toxic. The compounds of the invention may present different polymorphic forms, it is intended that the invention encompasses all such forms.

In an embodiment according to the invention the medicament is for the prophylaxis and/or treatment of Alzheimer's disease.

In an embodiment according to the invention the medicament is for the prophylaxis and/or treatment of one or more disorders selected from the group consisting of diarrhoea, lipoprotein disorders, migraine, obesity, arthritis, hypertension, arrhythmia, ulcer, learning, memory and attention deficits, cognition disorders, neurodegenerative diseases, demyelinating diseases, addiction to drugs and chemical substances including cocaine, amphetamine, ethanol and nicotine; tardive diskinesia, ischemic stroke, epilepsy, stroke, stress, cancer or psychotic conditions, in particular depression, anxiety, psychosis or schizophrenia; inflammation, or autoimmune diseases.

In an embodiment according to the invention the medicament is for the prophylaxis and/or treatment of one or more disorders selected from the group consisting of elevated trigyceride levels, chylomicronemia, dysbetalipoproteinemia, hyperlipoproteinemia, hyperlipidemia, mixed hyperlipidemia, hypercholesterolemia, lipoprotein disorders, hypertriglyceridemia, sporadic hypertriglyceridemia, inherited hypertriglyceridemia and/or dysbetalipoproteinemia.

In another embodiment according to the invention the medicament is for the prophylaxis and/or treatment of one or more disorders selected from the group consisting of pain, preferably neuropathic pain, inflammatory pain or other pain conditions involving allodynia and/or hyperalgesia.

Said medicament may also comprise any combination of one or more of the compounds of general formula (I) given above, stereoisomers thereof, physiologically acceptable salts thereof or physiologically acceptable solvates thereof.

Another aspect of the present invention is the use of at least one compound of general formula (I) given above as suitable active substances, optionally in form of one of the stereoisomers, preferably enantiomers or diastereomers, a racemate or in form of a mixture of at least two of the stereoisomers, preferably enantiomers and/or diastereomers, in any mixing ratio, or a corresponding salt thereof, or a corresponding solvate thereof, and optionally one or more pharmaceutically acceptable excipients, for the preparation of a medicament for the modulation of sigma receptors, preferably for the prophylaxis and/or treatment of Alzheimer's disease.

The medicament according to the present invention may be in any form suitable for the application to humans and/or animals, preferably humans including infants, children and adults and can be produced by standard procedures known to those skilled in the art. The composition of the medicament may vary depending on the route of administration.

The medicament of the present invention may for example be administered parentally in combination with conventional injectable liquid carriers, such as water or suitable alcohols. Conventional pharmaceutical excipients for injection, such as stabilizing agents, solubilizing agents, and buffers, may be included in such injectable compositions. These medicaments may for example be injected intramuscularly, intraperitoneally, or intravenously,

Solid oral compositions (which are preferred as are liquid ones) may be prepared by conventional methods of blending, filling or tabletting. Repeated blending operations may be used to distribute the active agent throughout those compositions employing large quantities of fillers. Such operations are conventional in the art. The tablets may for example be prepared by wet or dry granulation and optionally coated according to the methods well known in normal pharmaceutical practice, in particular with an enteric coating.

The mentioned formulations will be prepared using standard methods such as those described or referred to in the Spanish and US Pharmacopeias and similar reference texts.

Medicaments according to the present invention may also be formulated into orally administrable compositions containing one or more physiologically compatible carriers or excipients, in solid or liquid form. These compositions may contain conventional ingredients such as binding agents, fillers, lubricants, and acceptable wetting agents. The compositions may take any convenient form, such as tablets, pellets, capsules, lozenges, aqueous or oily solutions, suspensions, emulsions, or dry powdered forms suitable for reconstitution with water or other suitable liquid medium before use, for immediate or retarded release.

The liquid oral forms for administration may also contain certain additives such as sweeteners, flavoring, preservatives, and emulsifying agents. Non-aqueous liquid compositions for oral administration may also be formulated, containing edible oils. Such liquid compositions may be conveniently encapsulated in e.g., gelatin capsules in a unit dosage amount.

The compositions of the present invention may also be administered topically or via a suppository.

The daily dosage for humans and animals may vary depending on factors that have their basis in the respective species or other factors, such as age, sex, weight or degree of illness and so forth. The daily dosage for humans may preferably be in the range from 1 to 2000, preferably 1 to 1500, more preferably 1 to 1000 milligrams of active substance to be administered during one or several intakes per day.

Another aspect of the present invention refers to a method for the prophylaxis and/or treatment of Alzheimer's disease, the method comprising administering to the subject at least one compound of general formula (I) as described above and optionally at least one further active substance and/or optionally at least one auxiliary substance to the subject.

Another aspect of the present invention refers to a method for the prophylaxis and/or treatment of diarrhoea, lipoprotein disorders, migraine, obesity, elevated trigyceride levels, chylomicronemia, dysbetalipoproteinemia, hyperlipoproteinemia, hyperlipidemia, mixed hyperlipidemia, hypercholesterolemia, lipoprotein disorders, hypertriglyceridemia, sporadic hypertriglyceridemia, inherited hypertriglyceridemia and dysbetalipoproteinemia, arthritis, hypertension, arrhythmia, ulcer, learning, memory and attention deficits, cognition disorders, neurodegenerative diseases, demyelinating diseases, addiction to drugs and chemical substances including cocaine, amphetamine, ethanol and nicotine; tardive diskinesia, ischemic stroke, epilepsy, stroke, stress, cancer or psychotic conditions, in particular depression, anxiety or schizophrenia; inflammation, or autoimmune diseases, the method comprising administering to the subject at least one compound of general formula (I) as described above and optionally at least one further active substance and/or optionally at least one auxiliary substance to the subject.

A preferred embodiment of the present invention refers to a method for the prophylaxis and/or treatment of elevated trigyceride levels, chylomicronemia, dysbetalipoproteinemia, hyperlipoproteinemia, hyperlipidemia, mixed hyperlipidemia, hypercholesterolemia, lipoprotein disorders, hypertriglyceridemia, sporadic hypertriglyceridemia, inherited hypertriglyceridemia and/or dysbetalipoproteinemia.

A preferred other aspect of the invention refers to a production process using the Pauson-Khand Reaction, characterized in that the additive used in the Pauson-Khand Reaction is either
- Trioctyl phosphine oxide or
- Cyclopentylamine,
preferably trioctyl phosphine oxide.

Neither of these additives has been used as additives for Pauson-Khand reactions but gave surprisingly good results.

As stated above the Pauson-Khand reaction is a well-known chemical reaction known from textbooks to anyone skilled in the art. References include the following which are all included by reference into this description:
- Coordination Chemistry Reviews, 188 (1999), 297-341;
- Tetrahedron, 56 (2000), 3263-3283;
- Synlett 1999, 6, 768-770;
- Chem. Eur.J. 2001, 7 (8), 1589-1595;
- Journal of Organometallic Chemistry, 354 (1988) 233-242;
- Synlett 1999, 6, 771-773;
- Org. Biomol. Chem., 2005, 3, 2396-2398.

All the reaction conditions for the Pauson-Khand Reaction described above for the first aspect of the invention (the process for producing compounds of general formula I) are (if necessary modified) applicable for this reaction as well. This covers especially, that
- the reaction is done in an organic solvent and/or
- the reaction temperature is between 20° C and 120° C, preferably between 60° C and 100° C, most preferably between 75° C and 95° C and/or
- the reaction time is between 1h h and 48h, preferably between 12h and 24h and/or
- Co₂(CO)₈ is added at any time during this reaction and/or
- the organic solvent is halogenated and/or aliphatic, preferably is halogenated and aliphatic, most preferably is 1,2-dichloroethane and/or
- the reaction temperature is between 50° C and 120° C, preferably between 60° C and 90° C, most preferably at between 75° C and 85° C or at room temperature and/or
- the reaction time is between 6h and 48h, preferably between 12h and 36h, most preferably between 18h and 24h.

The present invention is illustrated below with the aid of examples. These illustrations are given solely by way of example and do not limit the general spirit of the present invention.

### EXAMPLES

**General Procedure for the synthesis of Pauson-Khand adducts.** To a solution of the acetylene (1.1 eq) (of general formula III (see above)) in 1,2-dichloroethane, was added Co₂(CO)₈ (1.1 eq) and the mixture was stirred 2 hours at room temperature. A solution of the pyrroline (1 eq) (of general formula II (see above and different examples 0 below)) in 1,2-dichloroethane and the additive (dimethylsulfoxide or cyclohexylamine) (3.5 eq) were added and the mixture was heated at 83°C for 20 hours.

The reaction mixture was filtered through celite and washed with CH₂Cl₂. The filtrate was concentrated and the crude was purified by flash chromatography.

As further additives were successfully tested:
- Trioctyl phosphine oxide and
- Cyclopentylamine.

### Synthesis of intermediates (according to general formula II):

### Example 0: 1-Benzyl-3-pyrroline

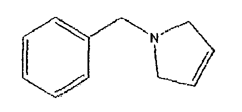

Was synthesized according to published methods with slight modifications: a) EP0985664, b) Synthetic Communications 13(13), 1117-1123 (1983).

To a solution of cis-1,4-dichloro-2-butene (0.76 g, 5.77 mmol) in anhydrous dichloromethane (4 ml) cooled at 5°C, benzylamine (3.75 g, 34.66 mol) was added dropwise. The mixture was stirred at 5°C for 10 min and after at r.t. 24 hours. The white solid was filtered and washed with dichloromethane. The filtrated was cooled at 0°C and HCl 37% (0.6 ml) was added. The resulting white solid was filtered and washed with dichloromethane. The filtrate was concentrated to give an orange oil that was purified by flash chromatography: silica gel, hexane:ethyl acetate (1:1) to afforded the product (0.76 g, 82%) as yellow oil.

**¹H NMR** (400 MHz, CDCl3): δ (ppm) 7.38-7.21 (m 5H), 5.78 (s, 2H), 3.80 (s, 3H), 3.81 (s, 2H), 3.48 (s, 4H). **¹³C NMR** (75 MHz, CDCl₃) δ (ppm) 139.67, 128.69, 128.34, 127.78, 126.96, 60.40, 59.70.

### Example 0-A: 1-(4-methoxybenzyl)-3-pyrroline.

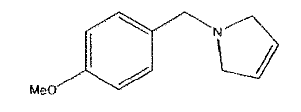

To a solution of 1,4-dimethylsulphonyl-2-butene (2,15 g, 11.64 mmol) in dichloromethane (40 ml), 4-methoxybenzylamine (6 ml, 44.24 mmol) was added dropwise and the solution was stirred 20 hours at r.t. The solid was filtrated and the filtrate was washed with water (2x30ml), dried over Na₂SO₄, filtered and concentrated. The crude was purified by flash chromatography: silica gel, gradient hexane:ethyl acetate 3:1 to 1:1. to afforded the product (1,4 g, 63%) as yellow oil.

**¹H NMR** (400 MHz, CDCl3): δ (ppm) 7.29 (d, J=8.5 Hz, 2H), 6.87 (d, J=8.5Hz, 2H), 5.79 (s, 2H), 3.80 (s, 3H), 3.76 (s, 2H), 3.48 (s, 4H). **¹³C NMR** (75 MHz, CDCl₃) δ (ppm) 158.83, 131.85, 129.83, 128.03, 114.31, 59.69, 59.56, 55.23.

### Example 0-B: 1-((S)-alpha-methylbenzyl)-3-pyrroline.

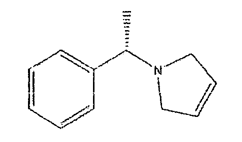

Was synthesized according to published methods with slight modifications: a) EP0985664, b) Synthetic Communications 34(23), 4421-4430 (2004).

To a solution of 1,4-dimethylsulphonyl-2-butene (0.52 g, 2.86 mmol) in dichloromethane (10 ml), (S)-alpha-methylbenzylamine (1.31 g, 10.87 mmol) was added and the solution was stirred 20 hours at r.t. The solid obtained was filtrated and the filtrate was washed with water (2x30ml), dried over Na₂SO₄, filtered and concentrated. The crude was purified by flash chromatography: silica gel, gradient hexane to hexane:ethyl acetate 1:1 to afforded the product (0.45 g, 90%) as yellow oil.

**¹H NMR** (400 MHz, CDCl3): δ (ppm) 7.42-7.25 (m, 5H), 5.80 (s, 2H), 3.53 (q, J=6.5Hz, 1H), 3.44 (m, 2H), 3.36 (m, 2H), 1.44 (d, J=6.5 Hz, 3H). **¹³C NMR** (75 MHz, CDCl₃) δ (ppm) 145.90, 128.47, 127.67, 127.28, 127.07, 65.30, 58.55, 23.50.

### Example 0-C :1-(Benzhydryl)-3-pyrroline

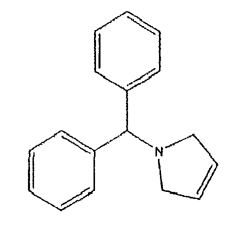

To a solution of aminodiphenylmethane (9.70 g, 51.46 mmol) in dichloromethane (6 ml) was added cis-1,4-dichloro-2-butene (1.18 g, 9.02 mmol) . The mixture was stirred at r.t. 20 hours. The white solid was filtered and washed with dichloromethane. The filtrated was cooled at 0°C and HCl 37% (2,5 ml) was added carefully and the suspension was stirred overnight. The resulting white solid was filtered and washed with dichloromethane and the filtrated was washed with saturated solution of NaHCO₃, water, dried over Na₂SO₄, filtered and concentrated. The crude was purified by flash chromatography: silica gel, gradient dichloromethane to dichloromethane:methanol 4% to afforded the product (0.90 g, 43%) as white solid. M.p. 90-91°C.

¹H NMR (400 MHz, CDCl3): δ (ppm) 7.54 (d, J=7Hz, 4H), 7.31 (t, J=7Hz, 4H), 7.19 (t, J=7Hz, 2H), 5.81 (s, 2H), 4.62 (s, 1H), 3.43 (s, 4H). ¹³C NMR (75 MHz, CDCl₃) δ (ppm) 144.12, 128.56, 127.62, 127.50, 127.00, 76.00, 59.37.

### Example 0-D: 1-(4-fluorobenzyl)-3-pyrroline

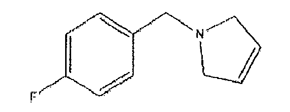

To a solution of cis-1,4-dichloro-2-butene (0.50 g, 3.8 mmol) in anhydrous dichloromethane (4 ml) cooled at 0°C, 4-fluorobenzylamine (2.94 g, 22.8 mol) was added dropwise. The mixture was stirred at 0°C for 10 min and then at r.t. 24 hours. The white solid was filtered and washed with dichloromethane. The filtrated was cooled at 0°C and HCl 10% was added until slightly acid pH. The resulting white solid was filtered and washed with dichloromethane. The filtrate was concentrated and the residue was purified by flash chromatography: silica gel, hexane:ethyl acetate (1:1) to afforded the product (353 m g, 52%) as yellow oil.

**¹H NMR** (400 MHz, CDCl3): δ (ppm) 7.31 (m, 2H), 6.99 (m, 2H), 5.78 (s, 2H), 3.77 (s, 2H), 3.46 (s, 4H). **¹³C NMR** (75 MHz, CDCl₃) δ (ppm) 161.91 (d, J_{CF}=243Hz), 135.41, 130.14 (d, J_{CF}=8Hz), 127.78 , 115.05 (d, J_{CF}=21Hz), 59.59, 59.52. **MS (ES+)** m/z: 178.1 (M+H⁺).

### Example 1: 2-benzyl-5-phenyl-1,2,3,3a-tetrahydrocyclopenta[c]pyrrol-4(6aH)-one.

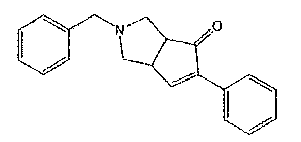

From phenylacetylene (5.0 g, 48.3 mmol), Co₂(CO)₈ (16.5 g, 48.3 mmol), 1-benzyl-3-pyrroline (7.0 g, 43.9 mmol), dimethylsulfoxide (12-0 g, 153.8 mmol) and 1,2-dichloroethane (200 ml). Purification: silica gel, gradient dichloromethane to dichloromethane:methanol 1%, afforded the product (5.9 g, 46%) as yellow oil.

**¹H NMR** (400 MHz, CDCl₃): δ (ppm) 7.72 (m, 2H), 7.65 (d, J=3Hz, 1H), 7.40-7.18 (m, 8H), 3.49-3.63 (AB system, 2H), 3.36 (m, 1H), 3.19 (d, J=9Hz, 1H), 2.94 (m, 1H), 2.83 (d, J=9Hz, 1H), 2.43 (t, J=9Hz, 1H), 2.37 (t, J=9Hz, 1H). **¹³C NMR** (75 MHz, CDCl₃) δ (ppm) 208.94, 159.74, 143.79, 138.30, 131.47, 128.45, 128.38, 128.34, 128.18, 58.91, 56.79, 55.89, 50.24, 42.58. **MS (El+)** m/z: 289.14 (M⁺).

### Example 2: 5-phenyl-2-((S)-1-phenylethyl)-1,2,3,3a-tetrahydrocyclopenta[c]pyrrol-4(6aH)-one.

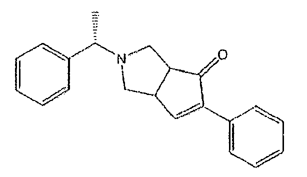

From phenylacetylene (37 mg, 0.35 mmol), Co₂(CO)₈ (121 mg, 0.35 mmol), 1-(S)-alpha-methyl-benzyl-3-pyrroline (56 mg, 0.32 mmol), dimethylsulfoxide (72 µl, 1.01 mmol) and 1,2-dichloroethane (2 ml). Purification: silica gel, gradient: dichloromethane to dichloromethane:methanol 1%, afforded the product (70 mg, 71%) as yellow oil, as mixture of two diastereomers (1:1).

**¹H NMR** (400 MHz, CDCl₃): δ (ppm) 7.76-7.71 (m, 4H), 7.70 (d, J=3Hz, 1H), 7.59 (d, J=3Hz, 1H), 7.44-7.16 (m, 16H), 3.41 (d, J=9Hz), 3.38 (m, 1H), 3.28 (m, 1H), 3.21 (q, J=6.5Hz, 1H), 3.18 (q, J=6.5Hz, 1H), 3.01 (d, J=9Hz, 1H), 2.96 (m, 1H), 2.94 (d, J=9Hz, 1H), 2.88 (m, 1H), 2.63 (d, J=9Hz, 1H), 2.42 (m, 2H), 2.31 (t, J=9Hz, 1H), 2.21 (t, J=9Hz, 1H), 1.30 (d, J=6.5Hz, 3H),1.29 (d, J=6.5Hz, 3H). **¹³C NMR** (75 MHz, CDCl₃) δ (ppm) 209.33, 208.88, 160.05, 159.82, 143.72, 143.60, 131.51, 128.89, 128.50, 128.37, 128.31, 128.27, 127.93, 127.92, 127.16, 127.14, 126.90, 126.88, 126.84, 126.20, 64.31, 64.13, 55.53, 55.28, 55.16, 54.44, 50.11, 50.03, 42.39, 42.27, 41.74, 23.07, 22.65. **MS (EI+)** m/z: 304.15 (M+H⁺).

### Example 3: 2-(4-methoxybenzyl)-5-phenyl-1,2,3,3a-tetrahydrocyclopenta[c]pyrrol-4(6aH)-one.

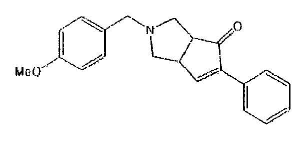

From phenylacetylene (930 mg, 8.93 mmol), Co₂(CO)₈ (3.0 g, 8.93 mmol), 1-(4-methoxy)-benzyl-3-pyrroline (1.3 g, 6.86 mmol), dimethylsulphoxide (2.1 g, 27.47 mmol) and 1,2-dichloroethane (30 ml). Reaction time 48 hours. Purification: silica gel, grad. dichloromethane to dichloromethane:melhanol 1%, afforded the product (985 mg, 45%) as yellow oil.

**¹H NMR** (400 MHz, CDCl₃): δ (ppm) 7.75-7.71 (m, 2H), 7.66 (d, J=3Hz, 1H), 7.42-7.31 (m, 3H), 7.12 (d, J=8.7Hz, 2H), 6.80 (d, J=8.7Hz, 2H), 3.78 (s, 3H), 3.57-3.44 (AB system, 2H), 3.37 (m, 1H), 3.17 (d, J=9Hz, 1H), 2.95 (m, 1H), 2.82 (d, J=9Hz, 1H), 2.42 (t, J=9Hz, 1H), 2.36 (t, J=9Hz, 1H), **¹³C NMR** (75 MHz, CDCl₃) δ (ppm) 209.07, 159.87, 158.53, 143.70, 131.46, 130.42, 129.56, 128.36, 127.14, 113.53, 58.25, 56.68, 55.77, 55.16, 50.22, 42.56. **MS (El+)** m/z: 319.15 (M⁺).

### Example 4: 2-benzyl-5-(4-fluorophenyl)-1,2,3,3a-tetrahydrocyclopenta[c]pyrrol-4(6aH)-one.

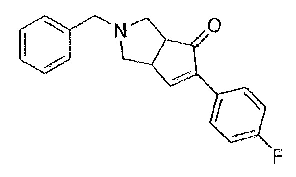

From 1-ethynyl-4-fluorobenzene (151 mg, 1.25 mmol), Co₂(CO)₈ (472 mg, 1.38 mmol), 1-benzyl-3-pyrroline (200 mg, 1.25 mmol), cyclohexylamine (436 mg, 4.39 mmol) and 1,2-dichloroethane (20 ml). Purification: silica gel, hexane:ethyl acetate (1:1), afforded the product (126 mg, 33%) as yellow oil.

**¹H NMR** (400 MHz, CDCl₃): δ (ppm) 7.75-7.71 (m, 2H), 7.63 (d, J=3Hz, 1H), 7.29-7.24 (m, 3H), 7.21 (m, 2H), 7.07 (m, 2H), 3.63-3.50 (AB system, 2H), 3.38 (m, 1H), 3.19 (d, J=9Hz, 1H), 2.94 (t, J=9Hz, 1H), 2.84 (d, J=9Hz, 1H), 2.43 (t, J=9Hz, 1H), 2.37 (t, J=9Hz, 1H). **¹³C NMR** (75 MHz, CDCl₃) δ (ppm) 208.92, 164.04, 159.37, 142.72, 138.28, 128.99, 128.91, 128.45, 128.24, 128.19, 126.94, 115.41, 115.19, 58.91, 56.80, 55.87, 50.18, 42.53. **HRMS (ES+)** m/z: calcd for C₂₀H₁₉NOF (M+H⁺): 308.1451; found: 308.1446.

### Example 5: 2-benzyl-5-(4-ethylphenyl)1,2,3,3a-tetrahydrocyclopenta[c]pyrrol-4(6aH)-one.

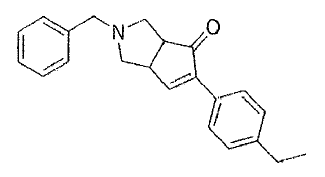

From 1-ethyl-4-ethynylbenzene (168 mg, 1.25 mmol), Co₂(CO)₈ (472 mg, 1.38 mmol), 1-benzyl-3-pyrroline (200 mg, 1.25 mmol), cyclohexylamine (436 mg, 4.39 mmol) and 1,2-dichloroethane (20 ml). Purification: silica gel, hexane:ethyl acetate (1:1), to afforded the product (40 mg, 10%) as yellow oil.

**¹H NMR** (400 MHz, CDCl₃) : δ (ppm) 7.65 (m, 2H), 7.61 (d, J=3Hz, 1H), 7.29-7.17 (m, 7H), 3.64-3.49 (AB system, 2H), 3.36 (m, 1H), 3.19 (d, J=9Hz, 1H), 2.94 (m, 1H), 2.83 (d, J=9Hz, 1H), 2.66 (q, J=8.5Hz, 2H), 2.44 (t, J=9Hz, 1H), 2.37 (t, J=9Hz, 1H), 1.24 (t, J=8.5Hz, 3H). **¹³C NMR** (75 MHz, CDCl₃) δ (ppm) 209.17, 159.03, 144.69, 143.73, 138.49, 128.90, 128.45, 128.19, 127.90, 127.16, 126.89, 58.98, 56.89, 56.03, 50.27, 42.59, 28.70, 15.54. **HRMS (ES+)** m/z: calcd for C₂₂H₂₄NO (M+H⁺): 318.1858; found: 318.1854.

### Example 6: 2-benzyl-5-(2-chlorophenyl)-1,2,3,3a-tetrahydrocyclopenta[c]pyrrol-4(6aH)-one.

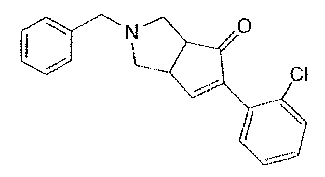

From 1-chloro-2-ethynylbenzene (171 mg, 1.25 mmol), Co₂(CO)₈ (472 mg, 1.38 mmol). 1-benzyl-3-pyrroline (200 mg, 1.25 mmol), dimethylsulphoxide (343 mg, 4.39 mmol) and 1,2-dichloroethane (20 ml). Purification: silica gel, hexane:ethyl acetate (1:1), afforded the product (128 mg, 32%) as yellow oil.

**¹H NMR** (400 MHz, CDCl3): δ (ppm) 7.68 (d, J=3Hz, 1H), 7.45-7.18 (m, 9H), 3.68-3.44 (AB system, 2H), 3.46 (m, 1H), 3.20 (d, J=9Hz, 1H), 2.94 (m, 1H), 2.86 (d, J=9Hz, 1H), 2.46 (t, J=9Hz, 1H), 2.38 (t, J=9Hz, 1H). **¹³C NMR** (75 MHz, CDCl₃) δ (ppm) 208.26, 163.70, 143.22, 138.65, 133.05, 130.80, 129.80, 129.25, 128.29, 128.18, 126.89, 126.49, 58.73, 56.95, 55.66, 49.17, 43.37. **HRMS (ES+)** m/z: calcd for C₂₀H₁₉NOCl (M+H⁺): 324.1155; found: 324.1140.

### Example 7: 2-benzyl-5-(4-chlorophenyl)-1,2,3,3a-tetrahydrocyclopenta[c]pyrrol-4(6aH)-one.

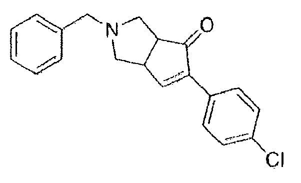

From 1-chloro-4-ethynylbenzene (171 mg, 1.25 mmol), Co₂(CO)₈ (472 mg, 1.38 mmol), 1-benzyl-3-pyrroline (200 mg, 1.25 mmol), dimethylsulphoxide (343 mg, 4.39 mmol), and 1,2-dichloroethane (20 ml) Purification: silica gel, hexane:ethyl acetate (1:1), afforded the product (148 mg, 36%) as yellow oil.

**¹H NMR** (400 MHz, CDCl₃) : δ (ppm) 7.74 (m, 1H), 7.69 (d, J=3Hz, 1H), 7.63 (m, 1H), 7.32-7.19 (m, 7H), 3.63-3.50 (AB system, 2H), 3.38 (m, 1H), 3.19 (d, J=9Hz, 1H), 2.95 (m, 1H), 2.85 (d, J=9Hz, 1H), 2.43 (t, J=9Hz, 1H), 2.38 (t, J=9Hz, 1H). **¹³C NMR** (75 MHz, CDCl₃) δ (ppm) 208.53, 160.63, 142.63, 138.30, 134.36, 133.21, 129.65, 128.48, 128.26, 127.24, 127.00, 125.33, 58.94, 56.87, 55.84, 50.27, 42.69. **HRMS (ES+)** m/z: calcd for C₂₀H₁₉NOCl (M+H⁺): 324.1155; found: 324.1144.

### Example 8: 2-benzyl-5-(3-chlorophenyl)-1,2,3,3a-tetrahydrocyclopenta[c]pyrrol-4(6aH)-one

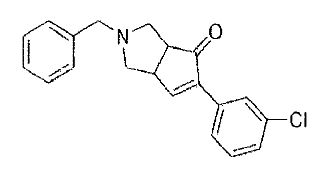

From 3-chloro-1-ethynylbenzene (171 mg, 1.25 mmol), Co₂(CO)₈ (472 mg, 1.38 mmol), 1-benzyl-3-pyrroline (200 mg, 1.25 mmol), dimethylsulphoxide (343 mg, 4.39 mmol), and 1,2-dichloroethane (20 ml). Purification: silica gel, hexane:ethyl acetate (1:1), afforded the product (143 mg, 35%) as yellow oil.

**¹H NMR** (400 MHz, CDCl₃): δ (ppm) 7.69 (m, 2H), 7.67 (d, J=3Hz, 1H), 7.35 (m, 2H), 7.26-7.18 (m, 5H), 3.64-3.50 (AB system, 2H), 3.37 (m, 1H), 3.19 (d, J=9Hz, 1H), 2.94 (m, 1H), 2.85 (d, J=9Hz, 1H), 2.44 (t, J=9Hz, 1H). 2.38 (t, J=9Hz, 1H). **¹³C NMR** (75 MHz, CDCl₃) δ (ppm) 208.78, 159.93, 142.67, 138.31, 134.36, 129.89, 128.59, 128.47, 128.22, 126.97, 58.92, 56.85, 55.86, 50.24, 42.64. **HRMS (ES+)** m/z: calcd for C₂₀H₁₉NOCl (M+H⁺): 324.1155; found: 324.1150.

### Example 9: 2-benzyl-5-(4-methoxyphenyl)-1,2,3,3a-tetrahydrocyclopenta[c]pyrrol-4(6aH]-one.

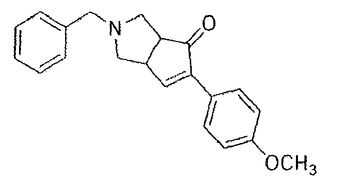

From 1-ethynyl-4-methoxybenzene (166 mg, 1.25 mmol), Co₂(CO)₈ (472 mg, 1.38 mmol), 1-benzyl-3-pyrroline (200 mg, 1.25 mmol), dimethylsulphoxide (343 mg, 4.39 mmol), and 1,2-dichloroethane (20 ml). Purification: silica gel, hexane:ethyl acetate (1:1), afforded the product (166 mg, 41%) as yellow oil.

**¹H NMR** (400 MHz, CDCl₃): δ (ppm) 7.71 (d, J=8.5Hz, 2H), 7.58 (d, J=3Hz, 1H), 7.29-7.19 (m, 5H), 6.92 (d, J=8.5Hz, 2H), 3.83 (s, 3H), 3.64-3.50 (AB system, 2H), 3.36 (m, 1H), 3.18 (d, J=9Hz, 1H), 2.94 (m, 1H), 2.83 (d, J=9Hz, 1H), 2.42 (t, J=9Hz, 1H), 2.36 (t, J=9Hz, 1H). **¹³C NMR** (75 MHz, CDCl₃) δ (ppm) 209.35, 159.78, 157.98, 143.09, 138.43, 128.47, 128.43, 128.19, 126.90, 124.12, 113.81, 59.00, 56.84, 56.08, 55.28, 50.26, 42.49. **HRMS (ES+)** m/z: calcd for C₂₁H₂₂NO₂ (M+H⁺): 320.1651; found: 320.1649.

### Example 10 :2-benzyl-5-(biphenyl-4-yl)-1,2,3,3a-tetrahydrocyclopenta[c]pyrrol-4(6aH)-one.

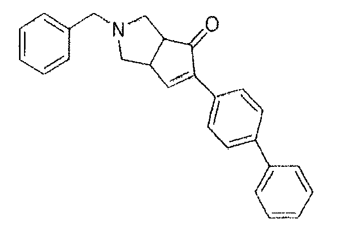

From 4-ethynyl-1,1'-biphenyl (246 mg, 1.38 mmol), Co₂(CO)₈ (472 mg, 1.38 mmol), 1-benzyl-3-pyrroline (200 mg, 1.25 mmol), dimethylsulphoxide (343 mg, 4.39 mmol), and 1,2-dichloroethane (20 ml). Purification: silica gel, hexane:ethyl acetate (2:1), afforded the product (163 mg, 35%) as yellow oil.

**¹H NMR** (400 MHz, CDCl3): δ (ppm) 7.82 (m, 2H), 7.71 (d, J=3Hz, 1H), 7.64-7.59 (m, 4H), 7.47-7.20 (m, 8H), 3.65-3.50 (AB system, 2H), 3.39 (m, 1H), 3.21 (d, J=9Hz, 1H), 2.97 (m, 1H), 2.86 (d, J=9Hz, 1H), 2.45 (t, J=9Hz, 1H), 2.39 (t, J=9Hz, 1H). **¹³C NMR** (75 MHz, CDCl₃) δ (ppm) 209.10, 159.65, 143.40, 141.21, 138.43, 131.80, 130.46, 128.78, 128.46, 128.22, 127.58, 127.41, 127.10, 127.05, 126.94, 58.98, 56.91, 50.32, 42.70. **HRMS (ES+)** m/z: calcd for C₂₆H₂₄NO (M+H⁺): 366.1858; found: 366.1848.

### Example 11: 2-benzyl-5-(4-tert-butylcarbamatephenyl)-1,2,3,3a-tetrahydrocyclopenta[c]pyrrol-4(6aH)-one

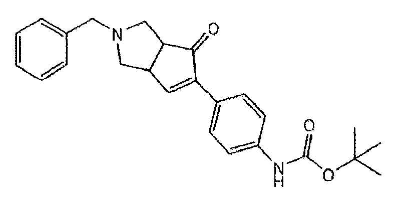

From 4-ethynyl-Boc-aniline (202 mg, 0.93 mmol), Co₂(CO)₈ (319 mg, 0.93 mmol), 1-benzyl-3-pyrroline (135 mg, 0.84 mmol), dimethylsulphoxide (232 mg, 2.96 mmol) and 1,2-dichloroethane (14 ml). Purification: silica gel, hexane:ethyl acetate (10:1), afforded the product (50 mg, 15%) as yellow oil.

**¹H NMR** (400 MHz, CDCl₃): δ (ppm) 7.62 (m, 2H), 7.53 (d, J=3Hz, 1H), 7.32-7.10 (m, 7H), 3.55-3.43 (AB system, 2H), 3.27 (m, 1H), 3.10 (d, J=9Hz, 1H), 2.85 (m, 1H), 2.76 (d, J=9Hz, 1H), 2.35 (t, J=9Hz, 1H), 2.29 (t, J=9Hz, 1H), 1.45 (s, 9H). **¹³C NMR** (75 MHz, CDCl₃) δ (ppm) 215.15, 158.64, 152.60, 143.05, 138.63, 138.41, 128.55, 128.27, 127.86, 126.95, 126.64, 126.21, 118.14, 81.20, 59.08, 56.81, 56.03, 50.34, 42.56, 28.38. **MS (ES+)** m/z: 405.21 (M+H⁺).

### Example 12 :2-benzyl-5-butyl-1,2,3,3a-tetrahydrocyclopenta[c]pyrrol-4(6aH]-one.

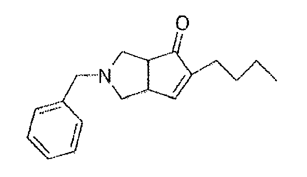

From 1-hexyne (26 mg, 0.31 mmol), Co₂(CO)₈ (118 mg, 0.34 mmol), 1-benzyl-3-pyrroline (50 mg, 0.31 mmol), dimethylsulphoxide (86 mg, 1.1 mmol) and 1,2-dichloroethane (2 ml). Purification: silica gel, hexane:ethyl acetate (1:1), afforded the product (45 mg, 53%) as yellow oil.

**¹H NMR** (400 MHz, CDCl₃): δ (ppm) 7.30-7.16 (m, 5H), 7.11 (m, 1H), 3.61-3.46 (AB system, 2H), 3.23 (m, 1H), 3.07 (d, J=9Hz, 1H), 2.75 (m, 1H), 2.71 (d, J=9Hz, 1H), 2.35 (t, J=9Hz, 1H), 2.28 (t, J=9Hz, 1H), 2.19 (m, 2H), 1.50 (m, 2H), 1.36 (m, 2H), 0.92 (t, J=7Hz, 3H). **¹³C NMR** (75 MHz, CDCl₃) δ (ppm) 211.29, 158.46, 146.98, 138.62, 128.38, 128.13, 126.84, 58.91, 56.67, 55.98, 49.19, 43.03, 29.83, 24.42, 22.28, 13.83. **MS (ES+)** m/z: 270.2 (M+H⁺).

### Example 13: 2-benzhydryl-5-phenyl-1,2,3,3a-tetrahydrocyclopenta[c]pyrrol-4(6aH)-one.

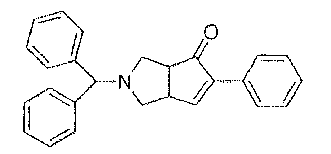

From phenylacetylene (22 mg, 0.21 mmol), Co₂(CO)₈ (90 mg, 0.26 mmol), 1-benzyl-3-pyrroline (50 mg, 0.31 mmol), dimethylsulphoxide (58 mg, 1.1 mmol) and 1,2-dichloroethane (2 ml). Purification: silica gel, hexane:ethyl acetate (10:1), afforded the product (16 mg, 21%) as yellow oil.

**¹H NMR** (400 MHz, CDCl₃): δ (ppm) 7.77 (m, 2H), 7.67 (d, J=3Hz, 1H), 7.55-7.35 (m, 4H), 7.32-7.11 (m, 9H), 4.17 (s, 1H), 3.34 (m, 1H), 3.16 (d, J=9Hz, 1H), 2.92 (m, 1H), 2.80 (d, J=9Hz, 1H), 2.31 (m, 2H). **¹³C NMR** (75 MHz, CDCl₃) δ (ppm) 208.91, 159.72, 144.01, 143.35, 142.99, 131.63, 128.51, 128.48, 127.30, 127.23, 127.02, 126.96, 74.38, 56.08, 55.46, 50.10, 42.35. **MS (ES+)** m/z: 366.2 (M+H⁺).

### Example 14: 2,5-dibenzyl-1,2,3,3a-tetrahydrocyclopenta[c]pyrrol-4(6aH)-one

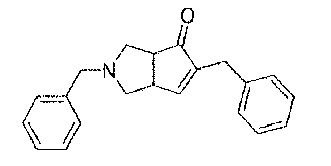

From 3-phenyl-1-propyne (37 mg, 0.31 mmol), Co₂(CO)₈ (107 mg, 0.31 mmol), 1-benzyl-3-pyrroline (50 mg, 0.31 mmol), dimethylsulphoxide (86 mg, 1.1 mmol) and 1,2-dichloroethane (2 ml). Purification: silica gel, hexane:ethyl acetate (10:1), afforded the product (44 mg, 46%) as yellow oil.

**¹H NMR** (400 MHz, CDCl3): δ (ppm) 7.33-7.15 (m, 10H), 7.01 (d, J=3Hz, 1H), 3.55 (AB system, 2H), 3.49 (AB system, 2H), 3.23 (m, 1H), 3.10 (d, J=9Hz, 1H), 2.79 (m, 1H), 2.70 (d, J=9Hz, 1H), 2.36 (t, J=9Hz, 1H), 2.25 (t, J=9Hz, 1H). **¹³C NMR** (75 MHz, CDCl₃) δ (ppm) 210.51, 160.12, 146.32, 138.92, 138.59, 128.80, 128.43, 128.36, 128.17, 126.88, 126.18, 58.84, 56.71, 55.77, 49.21, 43.13, 31.13. **MS (ES+)** m/z: 304.2 (M+H⁺).

### Example 15: 2-(4-fluorobenzyl)-5-phenyl-1,2,3,3a-tetrahydrocyclopenta[c]pyrrol-4(6aH)-one.

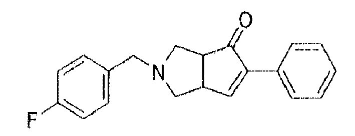

From phenylacetylene (29 mg, 0.28 mmol), Co₂(CO)₈ (107 mg, 0.31 mmol), 1-(4-fluoro)benzyl-3-pyrroline (50 mg, 0.28 mmol), dimethylsulphoxide (77 mg, 1.1 mmol) and 1,2-dichloroethane (2 ml). Purification: silica gel, hexane:ethyl acetate (5:1), afforded the product (41 mg, 47%) as yellow oil.

**¹H NMR** (400 MHz, CDCl₃): δ(ppm) 7.72 (d, J=7Hz, 2H), 7.65 (d, J=3Hz, 1H), 7.41-7.31 (m, 3H), 7.15 (m, 2H), 6.93 (t, J=8.5Hz, 2H), 3.58-3.45 (AB system, 2H), 3.37 (m, 1H), 3.17 (d, J=9Hz, 1H), 2.94 (m, 1H), 2.82 (d, J=9Hz, 1H), 2.40 (t, J=9Hz, 1H), 2,35 (t, J=9Hz, 1H). **¹³C NMR** (75 MHz, CDCl₃) δ (ppm) 208.95, 159.58, 143.74, 134.22, 131.50, 129.96, 129.85, 128.50, 128.35, 127.21, 115.14, 114.81, 58.32, 56.87, 56.02, 50.05, 42.48. **MS (ES+)** m/z: 308.2 (M+H⁺).

### Example 16: 2-benzyl-5-tert-butyl-1,2,3,3a-tetrahydrocyclopenta[c]pyrrol-4(6aH)-one.

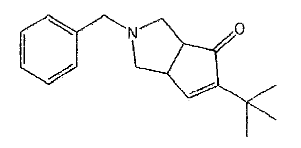

To a solution of 3,3-dimethyl-1-butyne (29 mg, 0.34 mmol) in 1,2-dichloroethane (1ml) cooled at 0°C was added Co₂(CO)₈ (118 mg, 0.34 mmol) and the mixture was stirred 15 min at 0°C and 40 minutes at room temperature. A solution of 1-benzyl-3-pyrroline (50 mg, 0.31 mmol) in 1,2-dichloroethane (1 ml) and dimethylsulfoxide (72 µl, 1.01 mmol) were added and the mixture was heated at 83°C for 20 hours. The reaction mixture was filtered through celite and washed with CH₂Cl₂. The filtrate was concentrated and the crude was purified by flash chromatography: silica gel, dichloromethane, to afforded the product (11 mg, 13%) as yellow oil.

**¹H NMR** (400 MHz, CDCl₃): δ (ppm) 7.29-7.17 (m, 5H), 7.08 (d, J=3Hz, 1H), 3.59-3.47 (AB system, 2H), 3.17 (m, 1H), 3.04 (d, J=9Hz, 1H), 2.72 (m, 1H), 2.68 (d, J=9Hz, 1H), 2.35 (t, J=9Hz, 1H), 2.30 (t, J=9Hz, 1H), 1.20 (s, 9H). **¹³C NMR** (75 MHz, CDCl₃) δ (ppm) 209.28, 156.44, 154.52, 128.27, 128.19, 126.88, 58.69, 58.78, 56.15, 50.23, 42.00, 31.72, 28.33, **MS (EI+)** m/z: 269.17 (M⁺).

### Example 17: ((R,S)-5,6)-2-benzyl-6-methyl-5-phenyl-tetrahydrocyclopenta[c]pyrrol-4(1H,2H,5H)-one.

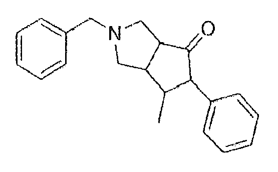

To a suspension of Cul (66 mg, 0.34 mmol) in diethylether (3 ml) under Ar, cooled at -50°C, MeLi was added and the mixture was stirred for 30 min at -50°C. After this time, a solution of the enone (100 mg, 0.34 mmol) in diethylether (2 ml) was added via cannula to the suspension at -50°C. Stirring was continued for 30 min, until no starting material was observed. The reaction mixture was quenched with a solution of NH₄Cl/NH₃ 10% and allowed to reach to r.t. Layers was separated and the aqueous phase was extracted with diethylether. The combined organic phases were washed with NH₄Cl/NH₃ 10% until blue colour disappears, dried over MgSO4, filtered and concentrated to dryness, to afforded the product (76 mg, 72%) as yellow oil.

**¹H NMR** (400 MHz, CDCl3), δ (ppm) 7.38-7.20 (m, 8H), 7.09 (m, 2H), 3.70-3.52 (AB system, 2H), 3.31 (d, J=9Hz, 1H), 3.13 (d, J=9Hz, 1H), 2.94 (m, 1H), 2.83 (d, J=9Hz, 1H), 2.42 (m, 1H), 2.32 (m, 3H), 2.15 (m, 1H), 1.09 (d, J=8.5Hz, 3H). **¹³C NMR** (75 MHz, CDCl₃) δ (ppm) 217.22, 137.21, 129.00, 128.93, 128.51, 128.37, 128.26, 128.19, 126.99, 126.90, 64.67, 59.36, 59.23, 56.46, 50.78, 45.30, 44.27, 18.90. **HRMS (ES+)** m/z: calcd for C₂₁H₂₄NO (M+H⁺): 306.1858; found: 306.1854.

### Example 18: ((R,S)-5,6)-2-benzyl-6-butyl-5phenyl-tetrahydrocyclopenta[c]pyrrol-4(1H,2H,5H)-one.

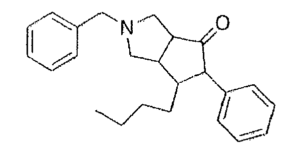

To a suspension of Cul (33 mg, 0.17 mmol) in diethylether (1.5 ml) under Ar, cooled at -50°C, n-BuLi was added and the mixture was stirred for 30 min at -50°C. After this time, a solution of the enone (50 mg, 0.17 mmol) in diethylether (1 ml) was added via cannula to the suspension at -50°C. Stirring was continued for 30 min, until no starting material was observed. The reaction mixture was quenched with a solution of NH₄Cl/NH₃ 10% and allowed to reach r.t. Layers was separated and the aqueous phase was extracted with diethylether. The combined organic phases were washed with NH₄Cl/NH₃ 10% until blue colour disappears, dried over MgSO₄, filtered and concentrated to dryness, to afforded the product (mg, %) as yellow oil.

**¹H NMR** (400 MHz, CDCl3): δ (ppm) 7.40-7.20 (m, 8H), 7.11 (m, 2H), 3.71-3.53 (AB system, 2H), 3.29 (d, J=9Hz, 1H), 3.20 (d, J=9Hz, 1H), 2.94 (t, J=9Hz, 1H), 2.83 (d, J=9Hz, 1H), 2.53 (m, 1H), 2.38 (m, 1H), 2.33 (t, J=9Hz, 1H), 2.14 (m, 1H), 1.58 (m, 1H), 1.40 (m, 1H), 1.20-1.11 (m, 4H), 0.78 (t, J=7Hz, 3H). **¹³C NMR** (75 MHz, CDCl₃) δ (ppm) 217.20, 137.10, 129.02,128.50, 128.35, 128.27, 126.95, 63.65, 60.96, 59.18, 56.52, 50.99, 49.12, 43.33, 34.87, 29.20, 22.87, 13.89. **MS (ES+)** m/z: 348.2 (M+H⁺).

### BIOLOGICAL ACTIVITY

Some representative compounds of the invention were tested for their activity as sigma (sigma-1 and sigma-2) inhibitors. The following protocols were followed:
Sigma-1
   Brain membrane preparation and binding assays for the σ1-receptor were performed as described (DeHaven-Hudkins et al., 1992) with some modifications. In brief, guinea pig brains were homogenized in 10 vols. (w/v) of Tris-HCl 50 mM 0.32 M sucrose, pH 7.4, with a Kinematica Polytron PT 3000 at 15000 r.p.m. for 30 s. The homogenate was centrifuged at 1000g for 10 min at 4°C and the supernatants collected and centrifuged again at 48000g for 15 min at 4°C. The pellet was resuspended in 10 volumes of Tris-HCl buffer (50 mM, pH 7.4), incubated at 37°C for 30 min, and centrifuged at 48000g for 20 min at 4°C. Following this, the pellet was resuspended in fresh Tris-HCl buffer (50 mM, pH 7.4) and stored on ice until use.
   Each assay tube contained 10 µL of [³H](+)-pentazocine (final concentration of 0.5 nM), 900 µL of the tissue suspension to a final assay volume of 1 mL and a final tissue concentration of approximately 30 mg tissue net weight/mL. Non-specific binding was defined by addition of a final concentration of 1 µM haloperidol. All tubes were incubated at 37°C for 150 min before termination of the reaction by rapid filtration over Schleicher & Schuell GF 3362 glass fibre filters [previously soaked in a solution of 0,5% polyethylenimine for at least 1 h]. Filters were then washed with four times with 4 mL of cold Tris-HCl buffer (50 mM, pH 7.4). Following addition of scintillation cocktail, the samples were allowed to equilibrate overnight. The amount of bound radioactivity was determined by liquid scintillation spectrometry using a Wallac Winspectral 1414 liquid scintillation counter. Protein concentrations were determined by the method of Lowry et al. (1951).
**Sigma-2**
   Binding studies for σ2-receptor were performed as described (Radesca et al., 1991) with some modifications. In brief, brains from sigma receptor type I (σ1) knockout mice were homogenized in a volume of 10 mL/g tissue net weight of ice-cold 10 mM Tris-HCl, pH 7.4, containing 320 mM sucrose (Tris-sucrose buffer) with a Potter-Elvehjem homogenizer (10 strokes at 500 r.p.m.) The homogenates were then centrifuged at 1000g for 10 min at 4°C, and the supernatants were saved. The pellets were resuspended by vortexing in 2 mL/g ice-cold Tris-sucrose buffer and centrifuged again at 1000g for 10 min. The combined 1000g supernatants were centrifuged at 31000g for 15 min at 4°C. The pellets were resuspended by vortexing in 3 mL/g 10 mM Tris-HCl, pH 7.4, and the suspension was kept at 25°C for 15 min. Following centrifugation at 31000g for 15 min, the pellets were resuspended by gentle Potter Elvehjem homogenization to a volume of 1.53 mL/g in 10 mM Tris-HCl pH 7.4.
   The assay tubes contained 10 µL of [³H]-DTG (final concentration of 3 nM), 400 µL of the tissue suspension (5.3 mL/g in 50 mM Tris-HCl, pH 8.0) to a final assay volume of 0.5 mL. Non-specific binding was defined by addition of a final concentration of 1 µM haloperidol. All tubes were incubated at 25°C for 120 min before termination of the reaction by rapid filtration over Schleicher & Schuell GF 3362 glass fibre filters [previously soaked in a solution of 0,5% polyethylenimine for at least 1 h]. Filters were washed with three times with 5 mL volumes of cold Tris-HCl buffer (10 mM, pH 8.0). Following addition of scintillation cocktail samples were allowed to equilibrate overnight. The amount of bound radioactivity was determined by liquid scintillation spectrometry using a Wallac Winspectral 1414 liquid scintillation counter. Protein concentrations were determined by the method of Lowry et al. (1951).

### References

DeHaven-Hudkins, D. L., L.C. Fleissner, and F. Y. Ford-Rice, 1992, "Characterization of the binding of [3H](+)pentazocine to σ recognition sites in guinea pig brain", Eur. J. Pharmacol. 227, 371-378.
Radesca, L., W.D. Bowen, and L. Di Paolo, B.R. de Costa, 1991, Synthesis and Receptor Binding of Enantiomeric N-Substituted cis-N-[2-(3,4-Dichlorophenyl)ethyl]-2-(1-pyrrolidinyl)cyclohexylamines as High-Affinity σ Receptor Ligands, J. Med. Chem. 34, 3065-3074.
Langa, F., Codony X., Tovar V., Lavado A., Giménez E., Cozar P., Cantero M., Dordal A., Hernández E., Pérez R., Monroy X., Zamanillo D., Guitart X., Montoliu LI., 2003, Generation and phenotypic analysis of sigma receptor type I (Sigma1) knockout mice, European Journal of Neuroscience, Vol. 18, 2188-2196.
Lowry, O.H., N.J. Rosebrough, A.L. Farr, and R.J. Randall, 1951, Protein measurement with the Folin phenol reagent, J. Biol. Chem, 193, 265.

Some of the results obtained are shown in table (I).

**Table (I)**

| Example | % Binding σ1 10⁻⁷M | % Binding σ1 10⁻⁸M |
|---|---|---|
| 1 | 79.4 | 46.8 |
| 2 | 40.1 | 38 |
| 3 | 47.3 | 11.3 |
| 4 | 88.9 | 57.4 |
| 5 | 102.3 | 54 |
| 6 | 92.7 | 45.5 |
| 7 | 67.5 | 46.9 |
| 8 | 104.1 | 68.3 |
| 9 | 97.5 | 73.4 |
| 10 | 57.0 | 34.2 |
| 11 | 23.3 | 17.7 |
| 12 | 101.7 | 53.2 |
| 13 | 27.6 | 4.8 |
| 14 | 85.7 | 31.0 |
| 15 | 71.7 | 29.8 |
| 16 | 54.1 | 33.3 |
| 17 | 106.6 | 82 |
| 18 | 72.3 | 28.2 |

## Claims

1. Process - using the Pauson-Khand Reaction - for the production of a substituted bicyclic tetrahydropyrrole compounds of general formula (I) 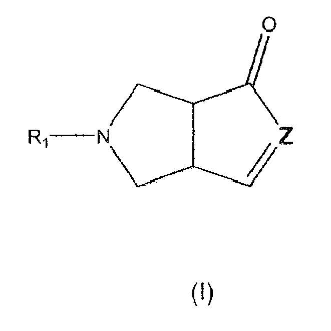 wherein
R¹ represents a hydrogen atom; an unbranched or branched, saturated or unsaturated, optionally at least mono-substituted aliphatic radical; a saturated or unsaturated, optionally at least mono-substituted, optionally at least one heteroatom as ring member containing cyclyl group, which may be condensed with an optionally at least mono-substituted mono- or polycyclic ring system; a branched or unbranched alkyl-cycloalkyl group in which either the alkyl group and/or the cycloalkyl group is optionally at least mono-substituted; a branched or unbranched, saturated or unsaturated, optionally at least mono-substituted alkyl-aryl group in which the aryl group may be condensed with another, optionally at least mono-substituted mono- or polycyclic ring system; a branched or unbranched, saturated or unsaturated, optionally at least mono-substituted alkyl-heterocyclyl group in which the heterocyclyl group is optionally condensed with another, at least mono-substituted mono- or polycyclic ring system; an optionally, at least mono-substituted benzhydryl group; a (C=O)-R² group; a (C=O)-OR³ group; a (SO₂)-R⁴ group; a (C=O)-NR⁵R^{5a} group;
Z represents C-R⁶; C-CHR⁷R^{7a}; a C-(C=O)-R⁸ group; a C-CH₂(SO₂)-R⁹ group; a C-CH₂(SO₂)-NR¹⁰R^{10a} group; or a C-(C=O)-NR¹⁰R^{10a} group;
R² , R³, R⁴, and R⁶ represent a hydrogen atom; a linear or branched, saturated or unsaturated, optionally at least mono-substituted aliphatic group; a saturated or unsaturated, optionally at least mono-substituted, optionally at least one heteroatom as ring member containing cyclyl group, which may be condensed with an optionally at least mono-substituted mono- or polycyclic ring system; a branched or unbranched alkyl-cycloalkyl group in which either the alkyl group and/or the cycloalkyl group is optionally at least mono-substituted; a branched or unbranched, saturated or unsaturated, optionally at least mono-substituted alkyl-aryl group in which the aryl group may be condensed with another, optionally at least mono-substituted mono- or polycyclic ring system; a branched or unbranched, saturated or unsaturated, optionally at least mono-substituted alkyl-heterocyclyl group in which the heterocyclyl group is optionally condensed with another, at least mono-substituted mono- or polycyclic ring system;
R⁵ and R^{5a} identical or different, represent a hydrogen atom; a linear or branched, saturated or unsaturated, optionally at least mono-substituted aliphatic group; a saturated or unsaturated, optionally at least mono-substituted, optionally at least one heteroatom as ring member containing cyclyl group, which may be condensed with an optionally at least mono-substituted mono- or polycyclic ring system; a branched or unbranched alkyl-cycloalkyl group in which either the alkyl group and/or the cycloalkyl group is optionally at least mono-substituted; a branched or unbranched, saturated or unsaturated, optionally at least mono-substituted alkyl-aryl group in which the aryl group may be condensed with another, optionally at least mono-substituted mono- or polycyclic ring system; a branched or unbranched, saturated or unsaturated, optionally at least mono-substituted alkyl-heterocyclyl group in which the heterocyclyl group is optionally condensed with another, at least mono-substituted mono- or polycyclic ring system;
R⁷ and R^{7a} identical or different, represent a hydrogen atom; a linear or branched, saturated or unsaturated, optionally at least mono-substituted aliphatic group; an unbranched or branched, saturated or unsaturated, optionally at least mono-substituted alkoxy radical; a saturated or unsaturated, optionally at least mono-substituted, optionally at least one heteroatom as ring member containing cyclyl group, which may be condensed with an optionally at least mono-substituted mono- or polycyclic ring system; a branched or unbranched alkyl-cycloalkyl group in which either the alkyl group and/or the cycloalkyl group is optionally at least mono-substituted; a branched or unbranched, saturated or unsaturated, optionally at least mono-substituted alkyl-aryl group in which the aryl group may be condensed with another, optionally at least mono-substituted mono- or polycyclic ring system; a branched or unbranched, saturated or unsaturated, optionally at least mono-substituted alkyl-heterocyclyl group in which the heterocyclyl group is optionally condensed with another, at least mono-substituted mono- or polycyclic ring system;
R⁸ and R⁹ represent a hydrogen atom; a linear or branched, saturated or unsaturated, optionally at least mono-substituted aliphatic group; an unbranched or branched, saturated or unsaturated, optionally at least mono-substituted alkoxy radical; a saturated or unsaturated, optionally at least mono-substituted, optionally at least one heteroatom as ring member containing cyclyl group, which may be condensed with an optionally at least mono-substituted mono- or polycyclic ring system; a branched or unbranched alkyl-cycloalkyl group in which either the alkyl group and/or the cycloalkyl group is optionally at least mono-substituted; a branched or unbranched, saturated or unsaturated, optionally at least mono-substituted alkyl-aryl group in which the aryl group may be condensed with another, optionally at least mono-substituted mono- or polycyclic ring system; a branched or unbranched, saturated or unsaturated, optionally at least mono-substituted alkyl-heterocyclyl group in which the heterocyclyl group is optionally condensed with another, at least mono-substituted mono- or polycyclic ring system;
R¹⁰ and R^{10a}, identical or different, represent a hydrogen atom; a linear or branched, saturated or unsaturated, optionally at least mono-substituted aliphatic group; an unbranched or branched, saturated or unsaturated, optionally at least mono-substituted alkoxy radical; a saturated or unsaturated, optionally at least mono-substituted, optionally at least one heteroatom as ring member containing cyclyl group, which may be condensed with an optionally at least mono-substituted mono- or polycyclic ring system; a branched or unbranched alkyl-cycloalkyl group in which either the alkyl group and/or the cycloalkyl group is optionally at least mono-substituted; a branched or unbranched, saturated or unsaturated, optionally at least mono-substituted alkyl-aryl group in which the aryl group may be condensed with another, optionally at least mono-substituted mono- or polycyclic ring system; a branched or unbranched, saturated or unsaturated, optionally at least mono-substituted alkyl-heterocyclyl group in which the heterocyclyl group is optionally condensed with another, at least mono-substituted mono- or polycyclic ring system;
optionally in form of one of the stereoisomers, preferably enantiomers or diastereomers, a racemate or in form of a mixture of at least two of the stereoisomers, preferably enantiomers and/or diastereomers, in any mixing ratio, or a corresponding salt thereof, or a corresponding solvate thereof;
wherein one substituted pyrroline compound of general formula (II) 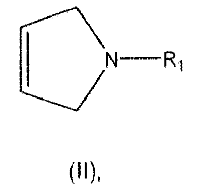 wherein R¹ has the meaning given above, is reacted - using a Pauson-Khand additive at any time during this reaction - with an acetylene compound of general formula (III), 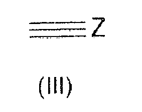 wherein Z has the meaning given above, to give compounds of general formula (I), 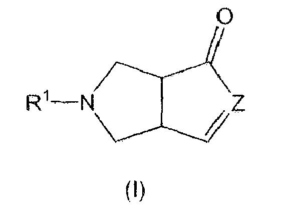 in which R¹ and Z have the meaning given above.

2. Process according to claim 1, wherein
• the reaction is done in an organic solvent and/or
• the reaction temperature is between 20° C and 120° C, preferably between 60° C and 100° C, most preferably between 75° C and 95° C and/or
• the reaction time is between 1 h and 48h, preferably between 12h and 24h and/or
• Co₂(CO)₈ is added at any time during this reaction.

3. Process according to claim 1, wherein in a preceeding
step A) in an organic solvent a compound of general formula (III), 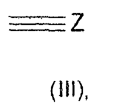 (wherein Z has the meaning according to claim 1), and Co₂(CO)₈ are dissolved/suspended and stirred between 0.5 and 4 h at a temperature between 4° C and 100° C, then in a
step B) a compound according to general formula II 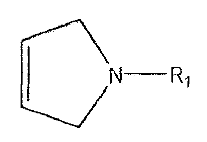 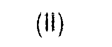 (wherein R¹ has the meaning according to claim 1) is added together with a Pauson-Khand additive and stirred for between 1 h and 48 h at between 20° C to 100 °C.

4. Process according to claim 3, wherein the compound according to general formula II and/or the Pauson-Khand additive is dissolved in an organic solvent before adding in Step B) to the compound arcording to general formula III.

5. Process according to any of claims 2, 3 or 4, wherein the organic solvent is halogenated and/or aliphatic, preferably is halogenated and aliphatic, most preferably is 1,2-dichloroethane.

6. Process according to any of claim 3, 4 or 5, wherein in step A)
• the reaction temperature is between 10° C and 40° C, preferably between 20° C and 30° C, most preferably at room temperature and/or
• the reaction time is between 0.75 h and 12 h, preferably between 1 h and 4h, most preferably between 1.5h and 2.5 h.

7. Process according to any of claim 3 to 6, wherein in step B)
• the reaction temperature is between 50° C and 120° C, preferably between 60° C and 90° C, most preferably at between 75° C and 85° C or at room temperature and/or
• the reaction time is between 6h and 48h, preferably between 12h and 36h, most preferably between 18h and 24h.

8. Process according to any of claim 1 to 7, wherein the compound according to general formula III and the compound according to general formula II are reacted in equal molar amounts ± 20%.

9. Process according to any of claim 2 to 7, wherein the compound according to general formula III, the compound according to general formula II and Co₂(CO)₈ are reacted in equal molar amounts ± 20%.

10. Process according to any of claim 1 to 7, wherein the Pauson-Khand additive is selected from
• Amine N-oxides (R₃NO)
• 4-methylmorpholine N-oxide
• Trimethylamine N-oxide
• Amines
• Cyclohexylamine
• Benzylamine
• α-methylbenzylamine
• Cyclooctylamine
• Phosphine-oxides (R₃PO)
• Tributylphosphine-oxide
• Sulphoxides (R-SO-R)
• Dimethylsulphoxide
• (-)-methyl-p-tolylsulfoxide
• Dialkylsulfides (R-S-R)
• n-butyl methyl sulphide
• methyl phenyl sulphide
• tert-butyl methyl sulphide
• dodecyl methyl sulphide
• 4-methylmorpholine N-oxide
• Methyl phenyl sulphide
• Cyclohexylamine
• Dimethylsulphoxide
• Trioctyl phosphine oxide
• Cyclopentylamine
• Trans-1,2-diaminocyclohexane
• 2-aminobutanol;
preferably from
• 4-methylmorpholine N-oxide
• Trimethylamine N-oxide
• Cyclohexylamine
• Benzylamine
• α-methylbenzylamine
• Cyclooctylamine
• Tributylphosphine-oxide
• Dimethylsulphoxide
• (-)-methyl-p-tolylsulfoxide
• n-butyl methyl sulphide
• methyl phenyl sulphide
• tert-butyl methyl sulphide
• dodecyl methyl sulphide
• 4-methylmorpholine N-oxide
• Methyl phenyl sulphide
• Cyclohexylamine
• Dimethylsulphoxide
• Trioctyl phosphine oxide
• Cyclopentylamine;
more preferably from
• 4-methylmorpholine N-oxide
• Methyl phenyl sulphide
• Cyclohexylamine
• Dimethylsulphoxide
• Trioctyl phosphine oxide
• Cyclopentylamine;
most preferably
• Cyclohexylamine
• Dimethylsulphoxide
• Trioctyl phosphine oxide
• Cyclopentylamine.

11. Process according to any of claim 1 to 10, wherein the Pauson-Khand additive is added in a molar amount equal to 1.5- to 5-times, preferably 3- to 4-times, the amount of the molar amount of the compound according to general formula III.

12. Process according to one or more of claims 1 to 16, wherein the produced compounds are selected from the group consisting of:
[1] 2-benzyl-5-phenyl-1,2,3,3a-tetrahydrocyclopenta[*c*]pyrrol-4(6a*H*)-one,
[2] 2-benzyl-5-phenyl-1,2,3,3a-tetrahydrocyclopenta[*c*]pyrrol-4(6a*H*)-one,
[3] 2-(4-methoxybenzyl)-5-phenyl-1,2,3,3a-tetrahydrocyclopenta[*c*]pyrrol-4(6a*H*)-one,
[4] 2-benzyl-5-(4-fluorophenyl)-1,2,3,3a-tetrahydrocyclopenta[*c*]pyrrol-4(6a*H*)-one,
[5] 2-benzyl-5-(4-ethylphenyl)1,2,3,3a-tetrahydrocyclopenta[*c*]pyrrol-4(6a*H*)-one,
[6] 2-benzyl-5-(2-chlorophenyl)-1,2,3,3a-tetrahydrocyclopenta[*c*]pyrrol-4(6a*H*)-one,
[7] 2-benzyl-5-(4-chlorophenyl)-1,2,3,3a-tetrahydrocyclopenta[*c*]pyrrol-4(6a*H*)-one,
[8] 2-benzyl-5-(3-chlorophenyl)-1,2,3,3a-tetrahydrocyclopenta[*c*]pyrrol-4(6a*H*)-one,
[9] 2-benzyl-5-(4-methoxyphenyl)-1,2,3,3a-tetrahydrocyclopenta[*c*]pyrrol-4(6a*H*]-one,
[10] 2-benzyl-5-(biphenyl-4-yl)-1,2,3,3a-tetrahydrocyclopenta[*c*]pyrrol-4(6a*H*)-one,
[11] 2-benzyl-5-(4-*tert*-butylcarbamatephenyl)-1,2,3,3a-tetrahydrocyclopenta[c]pyrrol-4(6aH)-one,
[12] 2-benzyl-5-butyl-1,2,3,3a-tetrahydrocyclopenta[*c*]pyrrol-4(6a*H*]-one,
[13] 2-benzhydryl-5-phenyl-1,2,3,3a-tetrahydrocyclopenta[*c*]pyrrol-4(6a*H*)-one,
[14] 2,5-dibenzyl-1,2,3,3a-tetrahydrocyclopenta[*c*]pyrrol-4(6a*H*)-one,
[15] 2-(4-fluorobenzyl)-5-phenyl-1,2,3,3a-tetrahydrocyclopenta[*c*]pyrrol-4(6a*H*)-one,
[16] 2-benzyl-5-*tert*-butyl-1,2,3,3a-tetrahydrocyclopenta[*c*]pyrrol-4(6a*H*)-one,
optionally in form of one of the stereoisomers, preferably enantiomers or diastereomers, a racemate or in form of a mixture of at least two of the stereoisomers, preferably enantiomers and/or diastereomers, in any mixing ratio, or a corresponding salt thereof, or a corresponding solvate thereof.

13. Production process using the Pauson-Khand Reaction, **characterized in that** the additive used in the Pauson-Khand Reaction is either
• Trioctyl phosphine oxide or
• Cyclopentylamine,
preferably trioctyl phosphine oxide.
